(19) Europäisches Patentamt European Patent Office Office européen des brevets

(11) **EP 4 374 379 B1**

(12) **EUROPEAN PATENT SPECIFICATION**

(45) Date of publication and mention of the grant of the patent:
**11.02.2026 Bulletin 2026/07**

(21) Application number: **22754836.9**

(22) Date of filing: **20.07.2022**

(51) International Patent Classification (IPC):
***G16H 10/40*** *(2018.01)*

(52) Cooperative Patent Classification (CPC):
**G16H 10/40; G16H 50/20;** G16H 40/20

(86) International application number:
**PCT/EP2022/070346**

(87) International publication number:
**WO 2023/001889 (26.01.2023 Gazette 2023/04)**

(54) **NON-INVASIVE METHOD FOR PREDICTING THE HEMOGLOBIN AND IRON CONTENT IN PRBC UNITS**

NICHTINVASIVES VERFAHREN ZUR VORHERSAGE DES HÄMOGLOBIN- UND EISENGEHALTS IN PRBC-EINHEITEN

PROCÉDÉ NON INVASIF PERMETTANT DE PRÉDIRE LA TENEUR EN HÉMOGLOBINE ET EN FER DANS DES UNITÉS PRBC

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2021 EP 21186556**

(43) Date of publication of application:
**29.05.2024 Bulletin 2024/22**

(73) Proprietor: **DRK-Blutspendedienst Baden-Württemberg-Hessen gGmbH 60528 Frankfurt am Main (DE)**

(72) Inventors:
• **NJOCK EPAH, Jeremy 64850 Schaafheim (DE)**
• **SCHÄFER, Richard 65779 Kelkheim (DE)**
• **SEIFRIED, Erhard 60320 Frankfurt (DE)**

(74) Representative: **Krauss, Jan SKM-IP PartGmbB Oberanger 45 80331 München (DE)**

(56) References cited:
WO-A1-2013/070592     WO-A1-2020/247702

• **CZUBAK-PROWIZOR KAMILA ET AL: "Red blood cell supernatant increases activation and agonist-induced reactivity of blood platelets", THROMBOSIS RESEARCH, ELSEVIER, AMSTERDAM, NL, vol. 196, 24 October 2020 (2020-10-24), pages 543 - 549, XP086358256, ISSN: 0049-3848, [retrieved on 20201024], DOI: 10.1016/J.THROMRES.2020.10.023**
• **RAJA ARSHAD ET AL: "Comparison of efficacy of packed red blood cell transfusion based on its hemoglobin content versus the standard transfusion practice in thalassemia major patients (HEMOCON study)", TRANSFUSION AND APHERESIS SCIENCE, ELSEVIER SCIENCE, LONDON, GB, vol. 59, no. 3, 27 January 2020 (2020-01-27), XP086154621, ISSN: 1473-0502, [retrieved on 20200127], DOI: 10.1016/ J.TRANSCI.2020.102736**

EP 4 374 379 B1

## Description

The present invention relates to a non-invasive computer-implemented method for determining and/or predicting total Hb (g) and/or total iron (mg) in a unit of packed red blood cells (pRBC). The method preferably further includes assigning the unit of pRBC to a group of pRBCs suitable for transfusion. Additional aspects relate to a data processing apparatus comprising means for carrying out the method, a computer program and a computer-readable medium.

## Background of the invention

The World Health Organization has acknowledged that blood transfusions are life-saving interventions with an essential role in patient management (1). Thus, providing hemoglobin (Hb) as oxygen carrier to prevent manifest anemia-related tissue hypoxia, the transfusion of packed red blood cells (pRBCs) is implemented in the therapy regimes of acute or chronic anemia due to blood loss, hematologic disorders, or after hematopoietic stem cell transplantation (HSCT) (2, 3). However, chronic Hb delivery comes with substantial side effects. This is highlighted by the fact that the accumulating heme iron load increases the transferrin saturation leading to the distribution of non-transferrin-bound iron (NTBI) throughout the body. Inducing reactive oxygen species, such as hydroxyl radicals and lipid peroxidative products, NTBIs cause damages in various organs, mainly in the heart, the liver and the endocrine glands (4). Subsequently, while saved from chronic hypoxemia due to provision of oxygen carrying Hb, patients depending on regular blood transfusions face the fatal risks of chronic iron overload (4). In fact, iron overload is associated with a decrease in overall survival in myelodysplastic patients and is associated with higher frequency of early and late transplant complications in HSCT (5). Notably, 20% of children with β-thalassemia major die by the age of 15 with the major cause of death being refractory myocardial failure due to iron overload, and 40% of young β-thalassemia patients die by the age of 20 if not treated with costly and side-effect prone chelation therapy (6-8).

Yet, precise iron monitoring remains difficult as measurements of serum ferritin cannot determine iron balance directly, does not follow high iron load levels linearly, and is affected by inflammation. Determination of liver iron content requires costly magnetic resonance imaging with limited availability, and liver biopsies come with risks of infections and bleedings [9].

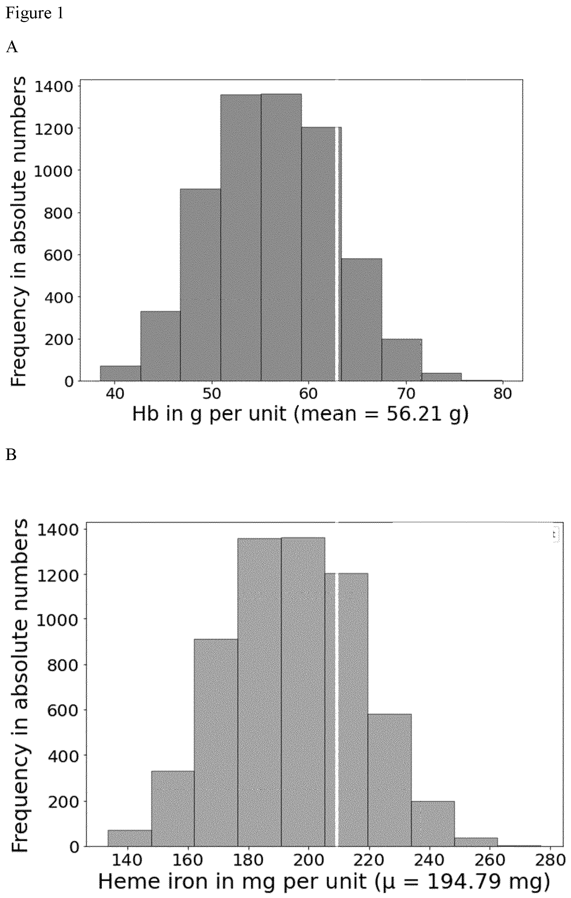

Packed RBCs vary in their total Hb content and, therefore, in their heme iron load from minimum to maximum up to 100% (9) (Figure 1). Even though some strategies have been developed to estimate transfusion-associated iron uptake, precise iron monitoring in chronically transfused patients remains difficult. For example, the measure of serum ferritin cannot determine the iron balance directly and it does not follow high iron load levels linearly. Moreover, serum ferritin levels can be affected independently of the iron status such as by inflammation. The determination of the liver iron content requires magnetic resonance imaging which is not universally available, expensive, and liver biopsies come with the risk of infections and bleedings (10).

Notably, to date the concentrations of neither the pharmaceutically active agent, *i.e.* Hb, nor of the potential noxa, *i.e.* heme iron, have been known for individual pRBC units and, thus, could not be considered for transfusions. This has led to the fact, that rules of thumb have been applied for clinical pRBC transfusions worldwide. For example, in pediatrics it is assumed that 5 mL pRBC unit / kg body weight will raise the patient's Hb concentration approximately about 1 g/dL (11), and in adults a similar result would be achieved by transfusing one complete pRBC unit (12) Thus, not only in the context of transfusion-associated chronic iron overload in a variety of diseases, but also considering patient blood management (PBM) and changing demographics with dramatic impact on blood supply, (13, 14) a more rational and evidence-based use of pRBCs is imperative.

International guidelines require a minimum of 35 g or 40 g Hb respectively per pRBC unit. However, such quality control testing can only be performed for a certain percentage of the pRBC production, and these products are discharged and thus, removed from the inventory. But how could the Hb content be determined for an individual pRBC unit that goes into the clinical inventory? Such a procedure cannot compromise the closed system in-line production and the pRBC unit's integrity due to risk of contamination.

Previously, attempts to find the "best fit" pRBC unit from the inventory for a given patient were carried out by developing an in-house blood banking software, that aimed to estimate the total Hb content of a pRBC unit simply by multiplying the volume (500 mL respectively 450 mL) of the whole blood donation with the measured fingertip Hb value of the donors (15, 16). However, a validation of the accuracy of these calculations with real Hb measurements was not reported (15, 16). In another approach a calculation-based estimation of the pRBC unit's Hb content was performed by additionally subtracting the blood volume that was lost during the production process. This study delivered a closer approximation to the overall Hb content distribution of the pRBC units, but did also not validate the Hb calculations to the Hb measurements of the respective units (9).

Big Data, machine learning (ML) and artificial intelligence promise to revolutionize medicine and support medical professionals in regards of decision making. Computers, being trained on specific ML algorithms, were enabled to support physicians, e.g. by classifying skin cancer images (17), or by predicting the progression from pre-diabetes to type 2 diabetes using routinely-collected data (18).

CN107356533A discloses a method for detecting the free hemoglobin content in a blood bag using a modulated light source measurement. A transmitted spectrum is collected, is normalized and included in a mathematical modeling to obtain the content of free hemoglobin.

Similarly, CN107367484A discloses a method for detecting the free hemoglobin content in a blood bag using a fluorescence excitation light source. A transmitted spectrum is collected, is normalized and included in a mathematical modeling to obtain the content of free hemoglobin.

Studies of the Hb content of PRBCs are provided by CZUBAK-PROWIZOR KAMILA ET AL: "Red blood cell supernatant increases activation and agonist-induced reactivity of blood platelets", THROMBOSIS RESEARCH, ELSEVIER, AMSTERDAM, NL, vol. 196, 24 October 2020, pages 543-549, and RAJA ARSHAD ET AL: "Comparison of efficacy of packed red blood cell transfusion based on its hemoglobin content versus the standard transfusion practice in thalassemia major patients (HEMOCON study)", TRANSFUSION AND APHERESIS SCIENCE, ELSEVIER SCIENCE, LONDON, GB, vol. 59, no. 3, 27 January 2020.

Thus, attempts were made previously to estimate the transfusion-associated iron uptake and the free hemoglobin content of red blood cell units. However, the accuracy of these concepts did not reach high levels, and robust validation data were not available.

In view of the above, it is an object of the present invention to provide improved methods and materials for predicting and detecting the total Hb and iron content of a pRBC unit. Other objects and advantages will become apparent to the person of skill when studying the present description of the present invention.

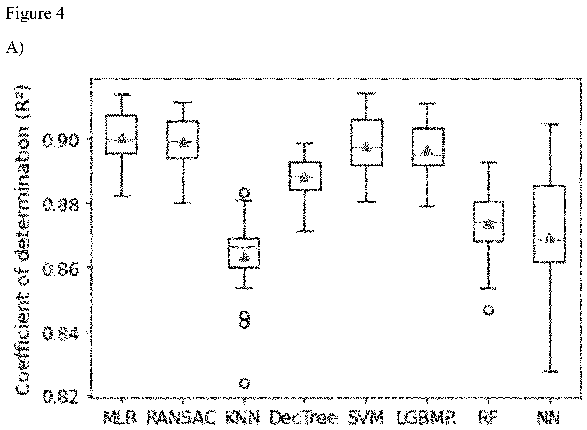

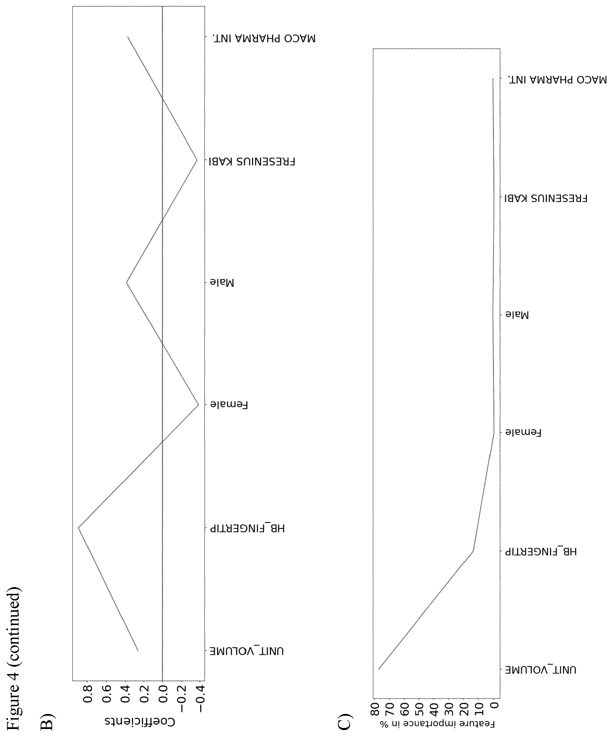

According to a first aspect of the present invention, the above object is solved by a method for predicting total Hb (g) in a unit of packed red blood cells (pRBC), comprising a) obtaining data for i) the volume (mL) of the unit, ii) the concentration of Hb Fingertip (g/dL), iii) the sex of the donor, and iv) the used type of blood bag system of the pRBC, and b) predicting total Hb (g) in said unit as provided by the following equation (see also Figure 4)

$$\beta0 + \beta1 * Unit\ volume\ (mL) + \beta2 * Hb\ Fingertip\left(\frac{g}{dL}\right) + \beta3 * xi3 + \beta4 * xi4$$
$$+ \beta5 * xi5 + \beta6 * xi6$$

wherein

$$\beta0 = (-33.97164897288266);\ \beta1 = 0.263;\ \beta2 = 0.893;$$

$$\beta3 = (-0.386);\ \beta4 = 0.386,$$

$$\beta5 = (-0.371)\ ;\ \beta6 = 0.371$$

xi3= 1 if female, else 0; xi4= 1 if male, else 0, xi5 = 1 if blood bag system CQ42271 FRESENIUS KABI DEUTSCHLAND GMBH else 0; xi6 = 1 if blood bag system LQT7248LC MACO PHARMA INT. GMBH else 0;

According to a second aspect of the present invention, the above object is solved by a method for predicting total iron (mg) in a unit of packed red blood cells (pRBC), comprising a) obtaining data for i) the volume (mL) of the unit, ii) the concentration of Hb Fingertip (g/dL), iii) the sex of the donor, and iv) the used type of blood bag system of the pRBC, and b) predicting total iron (mg) in said unit using the following equation

$$\frac{(\beta0 + \beta1 * Unit\ volume\ (mL) + \beta2 * Hb\ Fingertip\left(\frac{g}{dL}\right) + \beta3 * xi3 + \beta4 * xi4 + \beta5 * xi5 + \beta6 * xi6) * 4\ Fe * 55.845\frac{g}{mol}}{64.458\ \frac{g}{mmol}}$$

wherein

$$\beta0 = ((-33.97164897288266);\ \beta1 = 0.263;\ \beta2 = 0.893;$$

$$\beta3 = (-0.386);\ \beta4 = 0.386,\ \beta5 = -0.371;\ \beta6 = 0.371;$$

xi3= 1 if female, else 0; xi4= 1 if male, else 0, xi5 = 1 if blood bag system CQ42271FRESENIUS KABI DEUTSCHLAND

*GMBH else 0; xi6 = 1 if blood bag system LQT7248LC MACO PHARMA INT. GMBH else 0.*

According to a third aspect of the present invention, the above object is solved by a method for predicting total iron (mg) and total Hb (g) in a unit of packed red blood cells (pRBC), wherein the above methods according to the present invention are combined.

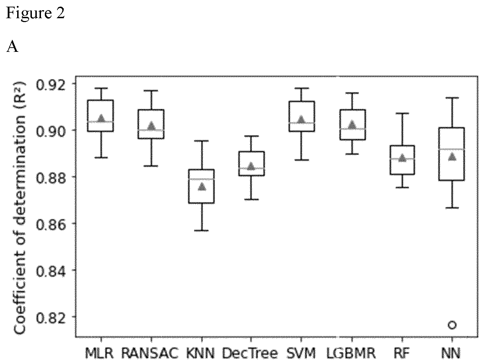

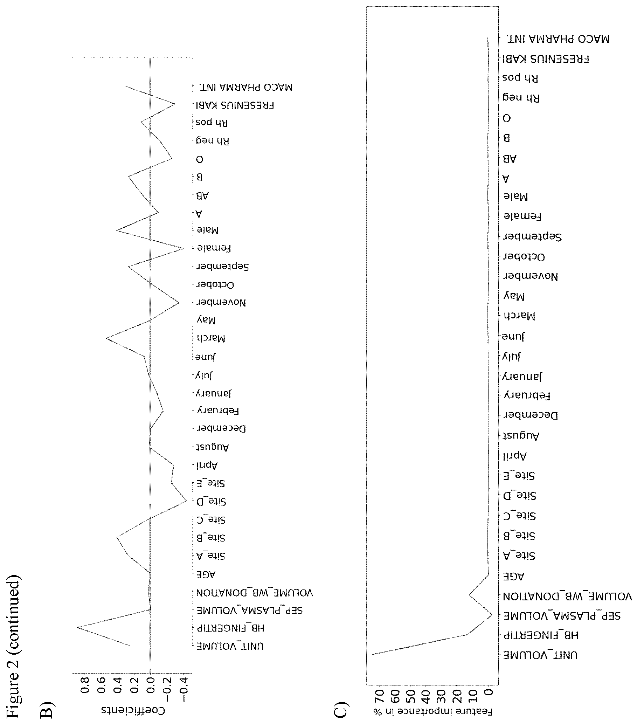

Machine learning algorithms and features such as total Hb content in gram, hematocrit (Hct) in percent, total unit volume in mL, extracted plasma volume in mL, production site, production date, donor age, donor sex, donor Hb in g/dL obtained from obligatory pre-collection fingertip testing, the volume of the whole blood collection in mL, used type of blood bag system, production machine and program were tested to predict Hb and iron content of pRBCs (Figure 2).

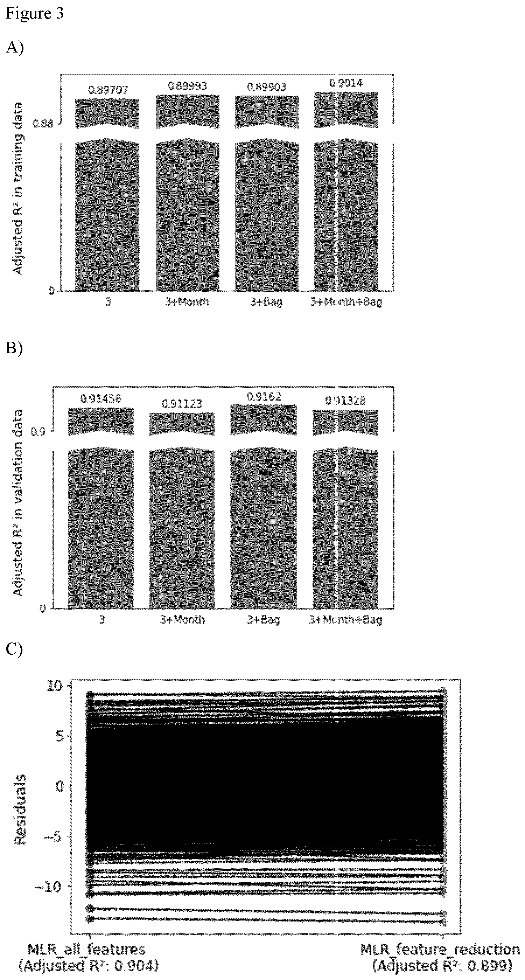

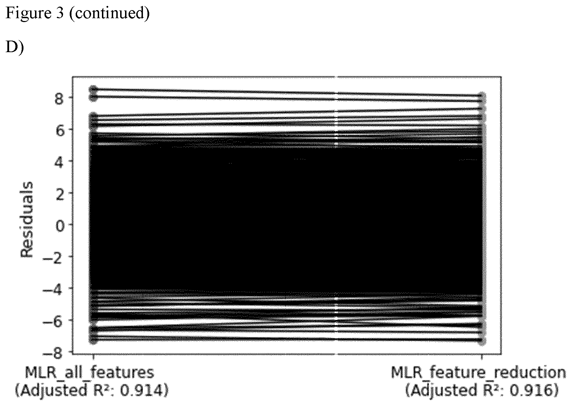

The inventors found that not all features were equally relevant for the Hb/iron prediction reducing the number of relevant features to 3 to 5, i.e. unit volume, Hb fingertip, sex, production month, and/or the used blood bag system (Figure 3).

For the Hb predictions considering all features the inventors observed seasonal variabilities with peaks in March, September and November (Figure 2), but the variable "season" was not identified as an indispensable component for Hb/Fe prediction in the validated cohort (Figure 3). Yet, seasonal variations may occur in various regions worldwide, and for other donor cohorts specific co-influencing factors might warrant further identification, e.g. based on local quality control data, to keep the accuracy of the predictions. Such, the inventor's approach that was validated with pRBCs from multiple production sites might be applicable to any blood bank worldwide with a sufficient amount of local adjustment including donor cohort-specific training and validation data. The inventors observed small differences between the different blood bag systems regarding their hemoglobin levels. Although the blood bag systems do not differ substantially in the composition of anticoagulant and RBC nutrient solution, they do differ in their valve systems, overall bag diameter, tube length as well as their tube diameter. This requires a specific compomate program for the respective blood bag system, which differs in the pressures applied and, thus, leads to variabilities in the production process and pRBC products.

Acknowledging the problem of precise hemoglobin and iron content prediction in individual red blood cell units, the inventors hypothesized that an approach that is not only based on donors Hb concentration and the volume of the whole blood donation could outperform the previous attempts delivering data accuracy being applicable for the clinic. Big Data, machine learning and artificial intelligence are revolutionizing medicine, and the inventors therefore tested if supervised machine learning approaches could provide reliable automatized predictions of hemoglobin and iron content in red blood cell units. The inventors trained machine learning (ML) algorithms and deep neural networks (DNNs) on donation and quality control data of 6,058 units that were collected between May 2018 and May 2020 and manufactured at five different production sites (Figure 2). Validating this concept in another cohort of 2,637 units, produced in the period of July 2020 to May 2021, the inventors verified the inventor's hypothesis that a non-invasive, automated process can precisely determine the hemoglobin and iron content in individual red blood cell units.

Preferred is the method according to the present invention, further comprising the step of c) assigning the unit of pRBC to a group of pRBCs suitable for transfusion or not and/or assigning the unit of pRBC as suitable or not for a given patient or group of patients, based on said predicting of total iron (mg) and/or total Hb (g).

According to a fourth aspect of the present invention, the above object is solved by a method according to the present invention, wherein said method is automated, such as, for example, performed by a robot.

According to a fifth aspect of the present invention, the above object is solved by a method according to the present invention, which is a computer-implemented method comprising receiving the data in step a) according to the present invention, followed by the predicting according to step b) according to the present invention.

According to a sixth aspect of the present invention, the above object is solved by a data processing apparatus comprising means for carrying out the method according to the present invention.

According to a seventh aspect of the present invention, the above object is solved by a computer program comprising instructions which, when the program is executed by a computer, cause the computer to carry out the method according to the present invention.

According to an eighth aspect of the present invention, the above object is solved by a computer-readable medium having stored there on the computer program according to the present invention.

As mentioned above, in a first aspect thereof, the present invention relates to a method for predicting total Hb (g) in a unit of packed red blood cells (pRBC), comprising a) obtaining data for i) the volume (mL) of the unit, ii) the concentration of Hb Fingertip (g/dL), iii) the sex of the donor, and iv) the used type of blood bag system of the pRBC, and b) predicting total Hb (g) in said unit using the following equation

$$\beta 0 + \beta 1 * Unit\ volume\ (mL) + \beta 2 * Hb\ Fingertip\left(\frac{y}{dL}\right) + \beta 3 * xi3 + \beta 4 * xi4 + \beta 5$$
$$* xi5 + \beta 6 * xi6$$

, wherein

$$\beta0 = (-33.\ 97164897288266);\ \beta1 = 0.263;\ \beta2 = 0.893;$$

$$\beta3 = (-0.386);\ \beta4 = 0.386,$$

$$\beta5 = (-0.371)\ ;\ \beta6 = 0.371$$

*xi3= 1 if female, else 0; xi4= 1 if male, else 0, xi5 = 1 if blood bag system CQ42271FRESENIUS KABI DEUTSCHLAND GMBH else 0; xi6 = 1 if blood bag system LQT7248LC MACO PHARMA INT. GMBH else 0*

In a second aspect thereof, the present invention relates to a method for predicting total iron (mg) in a unit of packed red blood cells (pRBC), comprising a) obtaining data for i) the volume (mL) of the unit, ii) the concentration of Hb Fingertip (g/dL), iii) the sex of the donor, and iv) the used type of blood bag system of the pRBC, and b) predicting total iron (mg) in said unit using the following equation

$$\frac{\left(\beta0 + \beta1 * Unit\ volume\ (mL) + \beta2 * Hb\ Fingertip\left(\frac{g}{dL}\right) + \beta3 * xi3 + \cdots + \beta6 * xi6\right) * 4\ Fe * 55.845\frac{g}{mol}}{64.458\ \frac{g}{mmol}}$$

wherein

$$\beta0 = (-33.\ 97164897288266);\ \beta1 = 0.263;\ \beta2 = 0.893;$$

$$\beta3 = (-0.\ 386);\ \beta4 = 0.\ 386,\ \beta5 = -0.371;\ \beta6 = 0.371$$

*xi3= 1 if female, else 0; xi4= 1 if male, else 0, xi5 = 1 if blood bag system CQ42271 FRESENIUS KABI DEUTSCHLAND GMBH else 0; xi6 = 1 if blood bag system LQT7248LC MACO PHARMA INT. GMBH else 0*

In an alternative embodiment thereof, the present invention relates to a method for predicting total Hb (g) in a unit of packed red blood cells (pRBC), comprising a) obtaining data for i) the volume (mL) of the unit, ii) the concentration of Hb Fingertip (g/dL), iii) and the sex of the donor, and b) predicting total Hb (g) in said unit using the following equation

$$\beta0 + \beta1 * Unit\ volume\ (mL) + \beta2 * Hb\ Fingertip\left(\frac{g}{dL}\right) + \beta3 * xi3 + \beta4 * xi4$$

, wherein

$$\beta0 = (-32.771212301579325);\ \beta1 = 0.254;\ \beta2 = 0.964;$$

$$\beta3 = (-0.426);\ \beta4 = 0.426$$

In yet another alternative embodiment thereof, the present invention relates to a method for predicting total iron (mg) in a unit of packed red blood cells (pRBC), comprising a) obtaining data for i) the volume (mL) of the unit, ii) the concentration of Hb Fingertip (g/dL), iii) the sex of the donor, and b) predicting total iron (mg) in said unit using the following equation

$$\frac{\left(\beta0 + \beta1 * Unit\ volume\ (mL) + \beta2 * Hb\ Fingertip\left(\frac{g}{dL}\right) + \beta3 * xi3 + \beta4 * xi4\right) * 4\ Fe * 55.845\frac{g}{mol}}{64.458\ \frac{g}{mmol}}$$

, wherein

$$\beta0 = (-32.771212301579325);\ \beta1 = 0.254;\ \beta2 = 0.964;$$

$$\beta 3 = (-0.426); \beta 4 = 0.426$$

In an alternative embodiment thereof, the present invention relates to a method for predicting total Hb (g) in a unit of packed red blood cells (pRBC), comprising a) obtaining data for i) the volume (mL) of the unit, ii) the concentration of Hb Fingertip (g/dL), iii) the sex of the donor, and iv) the month of donation of the pRBC, and b) predicting total Hb (g) in said unit using the following equation

$$\beta 0 + \beta 1 * Unit\ volume\ (mL) + \beta 2 * Hb\ Fingertip\left(\frac{g}{dL}\right) + \beta 3 * xi3 + \beta 4 * xi4\ ... + \beta 16 * xi16$$

wherein

$$\beta 0 = (-33.061629572325714); \beta 1 = 0.256; \beta 2 = 0.941;$$

$$\beta 3 = (-0.426); \beta 4 = 0.426;$$

$$\beta 5 = -0.124; \beta 6 = -0.187; \beta 7 = 0.069; \beta 8 = 0.097; \beta 9 = 0.058; \beta 10 = (-0.144); \beta 11 = (-0.133); \beta 12 = (0.874); \beta 13 = 0.165; \beta 14 = (-0.515); \beta 15 = (-0.222); \beta 16 = (0.063)$$

xi3= 1 if female, else 0; xi4= 1 if male, else 0.
xi5= 1 if April, else 0; xi6= 1 if August, else 0; xi7= 1 if December, else 0; xi8= 1 if February, else 0; xi9= 1 if January, else 0; xi10= 1 if July, else 0; xi11 = 1 if June, else 0; xi12= 1 ifMarch, else 0; xi13= 1 if May, else 0; xi14= 1 if November, else 0; xi15= 1 if October, else 0; xi16= 1 if September, else 0

In yet another alternative embodiment thereof, the present invention relates to a method for predicting total iron (mg) in a unit of packed red blood cells (pRBC), comprising a) obtaining data for i) the volume (mL) of the unit, ii) the concentration of Hb Fingertip (g/dL), iii) the sex of the donor, and iv) the month of donation of the pRBC, and b) predicting total iron (mg) in said unit using the following equation

$$\frac{\left(\beta 0 + \beta 1 * Unit\ volume\ (mL) + \beta 2 * Hb\ Fingertip\left(\frac{g}{dL}\right) + \beta 3 * xi3 + \beta 4 * xi4 + \cdots + \beta 16 * xi16\right) * 4\ Fe * 55.845\frac{g}{mol}}{64.458\ \frac{g}{mmol}}$$

wherein

$$\beta 0 = (-33.061629572325714); \beta 1 = 0.256; \beta 2 = 0.941;$$

$$\beta 3 = (-0.426); \beta 4 = 0.426;$$

$$\beta 5 = -0.124; \beta 6 = -0.187; \beta 7 = 0.069; \beta 8 = 0.097; \beta 9 = 0.058; \beta 10 = (-0.144); \beta 11 = (-0.133); \beta 12 = (0.874); \beta 13 = 0.165; \beta 14 = (-0.515); \beta 15 = (-0.222); \beta 16 = (0.063)$$

xi3= 1 iffemale, else 0; xi4= 1 if male, else 0.
xi5= 1 if April, else 0; xi6= 1 if August, else 0; xi7= 1 if December, else 0; xi8= 1 if February, else 0; xi9= 1 if January, else 0; xi10= 1 if July, else 0; xi11 = 1 if June, else 0; xi12= 1 if March, else 0; xi13= 1 if May, else 0; xi14= 1 if November, else 0; xi15= 1 if October, else 0; xi16= 1 if September, else 0

In an alternative embodiment thereof, the present invention relates to a method for predicting total Hb (g) in a unit of packed red blood cells (pRBC), comprising a) obtaining data for i) the volume (mL) of the unit, ii) the concentration of Hb Fingertip (g/dL), iii) the sex of the donor, iv) the month of donation and, v) the used type of blood bag system of the pRBC, and b) predicting total Hb (g) in said unit using the following equation

$$\beta 0 + \beta 1 * Unit\ volume\ (mL) + \beta 2 * Hb\ Fingertip\ \left(\frac{g}{dL}\right) + \beta 3 * xi3 + \beta 4 * xi4 + \ldots + \beta 18 * xi18$$

wherein

$$\beta 0 = (-34.07729847906681);\ \beta 1 = 0.263;\ \beta 2 = 0.88;$$

$$\beta 3 = (-0.391);\ \beta 4 = 0.391;$$

$$\beta 5 = -0.157;\ \beta 6 = -0.166;\ \beta 7 = 0.134;\ \beta 8 = (-0.012);\ \beta 9 = (-0.012);\ \beta 10 = (-0.117);\ \beta 11 = (-0.087);\ \beta 12 = (0.802);\ \beta 13 = 0.137;\ \beta 14 = (-0.467);\ \beta 15 = (-0.181);\ \beta 16 = (0.125);$$

$\beta 17 = (-0.331);\ \beta 18 = 0.331$
$xi3 = 1$ if female, else 0; $xi4 = 1$ if male, else 0.
$xi5 = 1$ if April, else 0; $xi6 = 1$ if August, else 0; $xi7 = 1$ if December, else 0; $xi8 = 1$ if February, else 0; $xi9 = 1$ if January, else 0; $xi10 = 1$ if July, else 0; $xi11 = 1$ if June, else 0; $xi12 = 1$ ifMarch, else 0; $xi13 = 1$ if May, else 0; $xi14 = 1$ if November, else 0; $xi15 = 1$ if October, else 0; $xi16 = 1$ if September, else 0; $xi17 = 1$ if blood bag system CQ42271 FRESENIUS KABI DEUTSCHLAND GMBH else 0; $xi18 = 1$ if blood bag system LQT7248LC MACO PHARMA INT. GMBH else 0

In yet another alternative embodiment thereof, the present invention relates to a method for predicting total iron (mg) in a unit of packed red blood cells (pRBC), comprising a) obtaining data for i) the volume (mL) of the unit, ii) the concentration of Hb Fingertip (g/dL), iii) the sex of the donor, and iv) the month of donation and, v) the used type of blood bag system of the pRBC, and b) predicting total iron (mg) in said unit using the following equation

$$\frac{\left(\beta 0 + \beta 1 * Unit\ volume\ (mL) + \beta 2 * Hb\ Fingertip\ \left(\frac{g}{dL}\right) + \beta 3 * xi3 + \beta 4 * xi4 + \cdots + \beta 18 * xi18\right) * 4\ Fe * 55.845\ \frac{g}{mol}}{64.458\ \frac{g}{mmol}}$$

wherein

$$\beta 0 = (-33.061629572325714);\ \beta 1 = 0.256;\ \beta 2 = 0.941;$$

$$\beta 3 = (-0.426);\ \beta 4 = 0.426;$$

$$\beta 5 = -0.124;\ \beta 6 = -0.187;\ \beta 7 = 0.069;\ \beta 8 = 0.097;\ \beta 9 = 0.058;\ \beta 10 = (-0.144);\ \beta 11 = (-0.133);\ \beta 12 = (0.874);\ \beta 13 = 0.165;\ \beta 14 = (-0.515);\ \beta 15 = (-0.222);\ \beta 16 = (0.063)$$

$xi3 = 1$ if female, else 0; $xi4 = 1$ if male, else 0.
$xi5 = 1$ if April, else 0; $xi6 = 1$ if August, else 0; $xi7 = 1$ if December, else 0; $xi8 = 1$ if February, else 0; $xi9 = 1$ if January, else 0; $xi10 = 1$ if July, else 0; $xi11 = 1$ if June, else 0; $xi12 = 1$ ifMarch, else 0; $xi13 = 1$ if May, else 0; $xi14 = 1$ if November, else 0; $xi15 = 1$ if October, else 0; $xi16 = 1$ if September, else 0; $xi17 = 1$ if blood bag system CQ42271 FRESENIUS KABI DEUTSCHLAND GMBH else 0; $xi18 = 1$ if blood bag system LQT7248LC MACO PHARMA INT. GMBH else 0

The inventors here present for the first time precise, non-invasive, Hb content/concentration predictions/determinations of individual pRBC units. Notably, the inventor's Hb prediction ML concept was validated by comparing the individual prediction to the measured Hb content of the respective pRBC unit. The inventor's concept therefore allows for the first time the declaration of hemoglobin and iron content of individual red blood cell units. Thus, for the first time, red blood cells can be dosed per their pharmaceutically active agent, and the iron uptake in chronically transfused patients can be precisely monitored, which enables clinicians to better control the life-limiting side effects and to optimize the costly iron chelation medication. Eventually, blood banks can, together with clinicians, adjust their transfusion regiments and are enabled to conduct true dose-efficacy studies in various clinical settings hereby promoting patient blood management to the next level.

In the context of the present invention, the term packed red blood cells (pRBCs) relates to red blood cells, in particular human blood cells that have been separated for blood transfusion. Units can be preferably produced from whole blood donations, usually varying around 500 mL, such as between 450 to 515 mL. They can be broadly collected, such from male or female European citizens, ranging in age from 18 to 73 years. Pre-donation fingertip Hb-levels may range from 12.5 g/dL up to 16.5 g/dL for women, and 13.5 g/dL up to 18.5 g/dL for men. Typically, the whole blood donations can be collected using standard equipment, such as with CompoFlow® System CQ42271 by Fresenius Kabi or LQT7248LC by Maco Pharma Int. GmbH, and processed with the CompoMat G5 Plus by Fresenius Kabi.

In the context of the present invention, the term production blood bag system of the pRBC relates to standard blood bag collection systems, preferably those that consist of blood donor bag, donor tube (consists of needle with needle cover), puncturable and nonsealable transfusion pot and clamp. The blood (donor) bag usually is a disposable biomedical transparent flexible polyvinyl chloride (PVC) container, designed to collect, process and store the whole blood and blood components. Preferably used are the blood bag collection systems CQ42271 produced by Fresenius Kabi and the blood bag system LQT7248LC by Maco Pharma Int. GmbH.

The current clinical practice for adults is to dose pRBCs in units. However, the transfusion effect, *i.e.* Hb increase in the patient's blood, varies significantly depending on several factors such as weight that correlates inversely to the Hb increase. The active agent of pRBCs, *i.e.* Hb, varies substantially between pRBC units, even of identical volume, or units that were manufactured from whole blood collections with the same volume from donors with the same fingertip Hb. Thus, specifying the total Hb content and Hb concentration of individual pRBC units are the key factors to define the actual dose of the drug. Also, the precise Hb-based dosing of pRBCs allows for the first time to conduct dose-effect studies for pRBC applications in different patient cohorts such as in surgery and hematology.

Preferred is then a method according to the present invention, further comprising the step of c) assigning the unit of pRBCs to a group of pRBCs suitable for transfusion or not, such as for the treatment of thalassemia, and/or assigning the unit of pRBCs as suitable or not for a given patient or group of patients, based on said predicting of total Hb (g), i.e. to be "fit" to a specific patient to be treated.

This the same for the prediction of total iron (mg). Preferred is thus a method according to the present invention, further comprising the step of c) assigning the unit of pRBCs to a group of pRBCs suitable for transfusion or not, such as for the treatment of thalassemia, and/or assigning the unit of pRBCs as suitable or not for a given patient or group of patients, based on said predicting of total iron (mg), i.e. to be "fit" to a specific patient to be treated.

Conveniently, the above methods can be combined, i.e. predicted is both total iron (mg) and total Hb (g) in a unit of packed red blood cells (pRBC) using a combination of the methods according to the present invention as herein. This combined method can then be used in a method according to the present invention, further comprising the step of c) assigning the unit of pRBCs to a group of pRBCs suitable for transfusion or not, such as for the treatment of thalassemia, and/or assigning the unit of pRBCs as suitable or not for a given patient or group of patients, based on said predicting of both total iron (mg) and total Hb (g), i.e. to be "fit" to a specific patient to be treated.

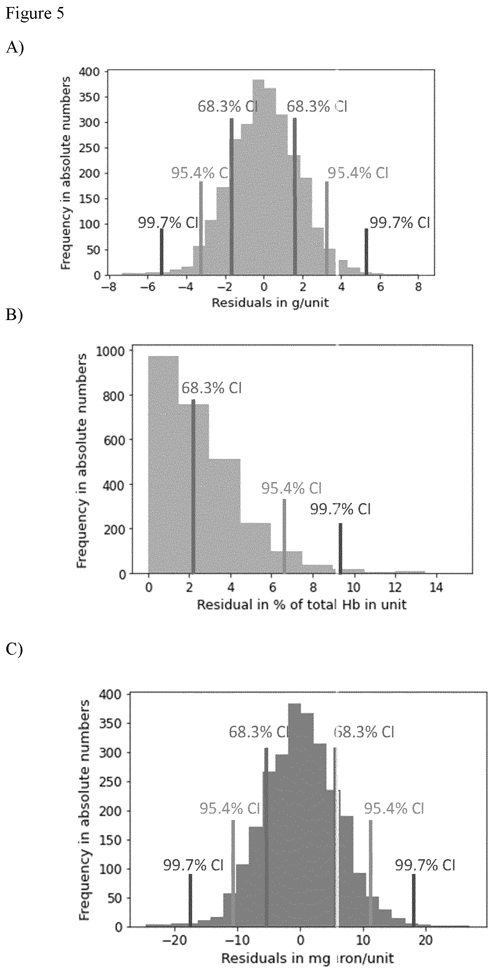

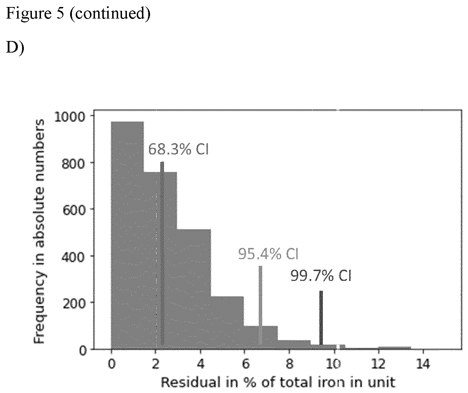

Applying an algorithm that was developed using a machine learning approach, the inventors identified relevant factors for hemoglobin and iron content in red blood cell units such as unit volume, hemoglobin concentration in blood sample from the fingertip taken prior to donation, sex of donor, separated plasma volume during production, and the used type of blood bag system. Alternatively or in addition to the type of blood bag system, the month of donation may be used as a factor in the algorithm (see above). The inventors have validated the inventor's approach with red blood cell units produced at multiple sites, and therefore the here presented concept should be applicable to any blood bank worldwide with a sufficient amount of local training and validation data. Advantageously, with the present method, the individual hemoglobin content of red blood cell units can be predicted at a mean accuracy of 97.45 %, 93.37% for the 95-CI and 91.33% accuracy for the 99.7-CI (Figure 5B, D). Therefore, preferred is the method according to the present invention, wherein the prediction accuracy of Hb has a mean accuracy of 0.05 g Hb and a 95% confidence interval of $\pm 3.54$ g Hb, and/or the iron content has a mean accuracy of 0.17 mg and a 95% confidence interval of about 12.27 mg in the units as included in the prediction (Figure 5A, C). In the context of the present invention, the term "about" shall mean +/- 10% of a given value, unless indicated otherwise.

In a preferred embodiment of the method according to the present invention, the method further comprises the step of determining the total iron (mg) and/or total Hb (g) in said unit or group of units of pRBCs. The determining can be basically preferably identical (i.e. is based on) to the prediction method, and then takes into account the CI of the method. Another alternative relates to an actual determination based on the analysis of the pRBC-unit, and the result may be used in order to further train the system as used for the present invention.

Another aspect of the present invention then relates to a method according to the present invention as described herein, wherein the data in step a), i.e. data for i) the volume (mL) of the unit or units as analyzed, ii) the concentration of Hb Fingertip (g/dL), iii) the sex of the donor for the unit, and iv) the type of blood bag system, and/or v) the month of donation is obtained by and/or received from a data recording device. These data are preferably taken from the information on the label of the unit(s) of pRBCs, such as, for example, by a bar code scanner reading a respective bar code.

Another aspect of the present invention then relates to a method according to the present invention as described herein, wherein said method is automated, such as, for example, performed by a robot. More preferably, the robot is controlled by a controlling unit. Automatization is particularly useful in larger collections of pRBC units, e.g. in a blood bank, where the system would be capable of assigning the best fit between the need(s) of a patient, and a unit or units of pRBCs as stored in the bank.

Another aspect of the present invention then relates to a method according to the present invention as described herein, wherein said method is a computer-implemented method, comprising receiving the data in step a), followed by the predicting according to step b). Preferably, the method furthermore puts out information regarding the prediction. Further preferred is a method, wherein said pRBC unit is furthermore assigned to a group of pRBCs suitable for transfusion or not, such as for the treatment of thalassemia, and/or assigning the unit of pRBCs as suitable or not for a given patient or group of patients, based on said predicting of total iron (mg) and/or total Hb (g), i.e. to be "fit" to a specific patient to be treated. The results of the prediction and/or the assignment may be stored in a data storage unit, and/or displayed or put out to another device(s).

Another aspect of the present invention then relates to a data processing apparatus comprising means for carrying out the method according to the present invention. Preferably, these means include means for controlling and receiving the data from recording device and/or means for controlling the robot as described above.

Another aspect of the present invention then relates to a computer program comprising instructions which, when the program is executed by a computer, cause the computer to carry out the method of the present invention. Finally, the present invention relates to a computer-readable medium having stored there on the computer program of the present invention.

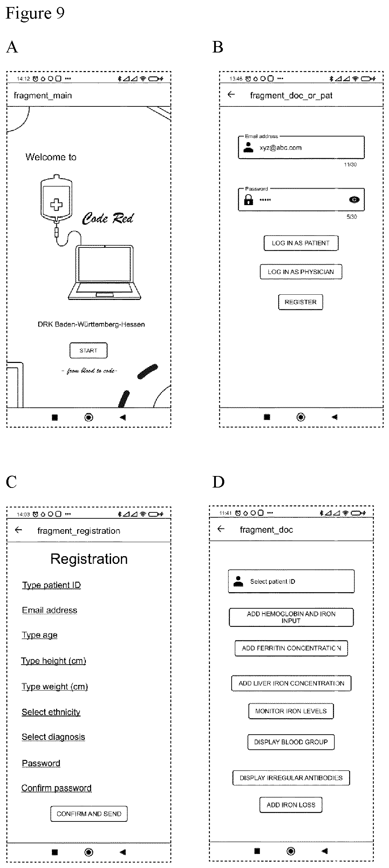

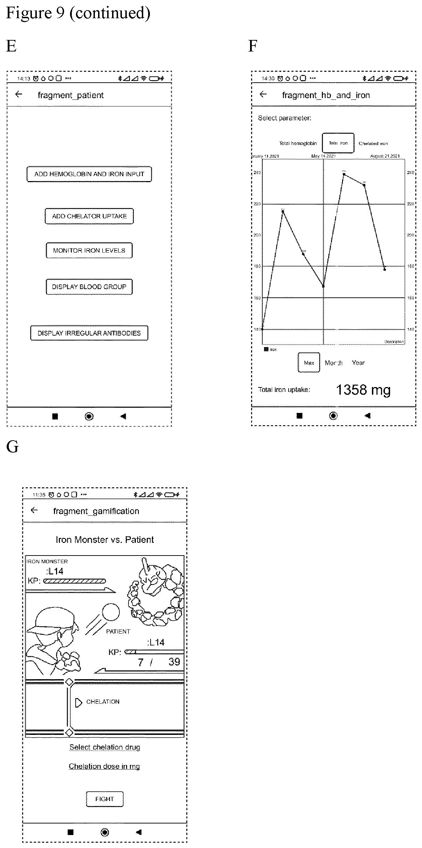

Another aspect of the present invention then relates to the computer program as above implemented in a smartphone application to facilitate iron monitoring for both physicians and patients (Figure 9). Forgetfulness and a lack of social support could affect compliance to oral medication in chronically ill pediatric patients. One possible future application of the inventor's method in pediatric patients could be in sickle cell disease (SCD), where many European countries, the U.S.A. and Canada implemented newborn SCD screening programs. The present method would not only allow to non-invasively monitor iron dosage and chelation therapy, but could also increase therapy adherence through gamification and fewer invasive procedures.

Blood donors could also benefit from the here presented approach. Protecting those is paramount for the entire health care system for ethical reasons and to ensure the supply of blood products. It is well documented that anemia from iron deficiency negatively effects learning and educational achievement. Notably, increased risk for iron deficiency can last up to a year after donation and frequent blood donations can lead to iron deficiency even in adults. A recent study reported accelerated iron stores and Hb depletion in teenage blood donors highlighting the need for improved measures to protect vulnerable young donors from donation-induced iron deficiency and iron deficiency anemia. Thus, advanced documenting and monitoring could increase donor safety and limit donation side effects.

As mentioned above, the current clinical practice for adults is to dose pRBCs in units. However, the transfusion effect, *i.e.* Hb increase in the patient's blood, varies significantly depending on several factors such as weight that correlates inversely to the Hb increase (42). Moreover, the inventors show here that the active agent of pRBCs, *i.e.* Hb, varies substantially between pRBC units (Figure 1). This is even true for units of identical volume, or units that were manufactured from whole blood collections with the same volume from donors with the same fingertip Hb. Thus, specifying the total Hb content and Hb concentration of individual pRBC units are the key factors to define the actual dose of the drug. Specifically, such precise Hb-based dosing would allow for the first time to conduct dose-effect studies for pRBC applications in different patient cohorts such as in surgery and hematology.

As per international consensus the implementation of PBM programs for various clinical scenarios warrants further high-quality research (43). Specifically, clinical recommendations for RBC transfusion thresholds and PBM program implementations, including computerized decision support systems, to improve RBC utilization are mainly based on low to moderate certainty in the evidence of effects (43). It is reasonable to hypothesize that the unknown individual Hb concentration in pRBC units substantially contributes to this current uncertainty. The here presented concept now allows the design of PBM studies considering accurate Hb dosing instead of neglecting the Hb concentrations of the applied pRBC units. Such precision transfusion medicine would not only supply the individual patient with the actually required Hb dose, but would also enable blood banks to more precisely manage and monitor their pRBC inventory. Such shift from units to actual Hb doses could also increase the general availability of pRBCs including O negative products. This is of particular importance as, due to the demographic changes, the future demand of RBC products could supersede the supply if blood centers would not adjust their operations (44).

Moreover, Hb-adjusted dosing could prevent unnecessary transfusions, hereby reducing the risk of infections, immunizations, wrong-patient blood transfusion errors and transfusion-associated circulatory overload (45), where specifically the latter comes in risk groups at a frequency of 1:12 or less (2).

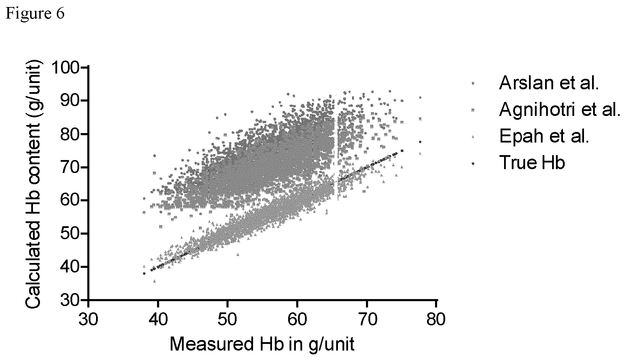

Thus, also other groups have thought about strategies to estimate the total Hb content in pRBC units, but they either used simplified calculations without comparing their estimations to the true Hb content (16), or compared their calculations only to the overall Hb content distribution of the analyzed pRBC cohort (9). Despite its limitations the first of these aforementioned studies showed that a transfusion regime considering Hb content estimations could decrease the numbers of transfused pRBC units for some patients (16). The low accuracy of the second study is specifically highlighted by the substantial (20%) difference between the standard deviation of the calculated Hb content per unit and the standard deviation of the actual Hb content (9). To the best of the inventor's knowledge the inventors here present for the first time precise, non-invasive, Hb content/concentration predictions of individual pRBC units. Notably, the inventor's Hb prediction ML concept was validated by comparing the individual prediction to the measured Hb content of the respective pRBC unit. Thus, the inventor's strategy is outperforming the previous concepts as highlighted by a more precise prediction accuracy (Figure 6, Table 2).

Machine learning-based systems have already demonstrated their potential to analyze and classify pRBC units for quality control, and to reveal so far unknown patterns (46). The special feature of dNNs is their ability to model non-linearity. Yet, for the prediction of Hb content in pRBC units ML algorithms which can model non-linear functions such as dNNs, RF and SVM were not superior to the MLR model (Figure 2, Figure 4). This suggests that the inventors were confronted with a linear multidimensional relationship in the inventor's framework.

The inventors found that more than every third (38%) of the here tested pRBC units substantially deviated from the mean Hb content (56.21 g). Given the annual production for Germany in 2017 (3.5 million pRBC units, (49)) these 38% represent the considerable amount of 1.3 million pRBC units.

Blood banks that could support clinicians to adjust their transfusion regiments would not only decrease the above mentioned transfusion risks, but would also lead to improved monitoring of transfusion associated iron uptake, which is highly variable from 6 to 41 mg per day (50). In patients with thalassemia who do not receive transfusions, iron absorption from the intestine, triggered by expansion of red cell precursors in the bone marrow, is increased, but transfusing RBCs to an Hb above 9 g/dL can avoid this expansion (50, 51). However, in transfusion-dependent thalassemia, the contribution of dietary iron to the total iron load is minor compared to the effect of pRBC transfusions which increase the iron stores to many times the norm unless chelation treatment is provided (50). The effects of iron chelation drugs have been studied for decades, and List *et al.* showed that chelation drug therapy can decrease the most toxic NTBI fraction, *i.e.* labile plasma iron (52). Referring to this hallmark study the inventors calculated, based on the minimal, average and maximal iron content of the pRBCs that the inventors investigated in the inventor's experiments, the respective daily iron load and the chelation drug doses and costs (Table 3). Hereby, the inventors show that it is possible to precisely adjust the chelation drug dose to the daily transfusion-related iron load and to better control the economic impact of this costly therapy.

Taken together, the inventor's concept allows to apply the required Hb dose with minimal iron burden, hereby also optimizing the chelation medication. This highlights the relevance of the inventor's findings for the clinical transfusion practice that should not only consider the Hb/iron-adjusted dosing, but must also consider the required matching parameters such as blood group and CMV status.

The here presented invention shows that, applying an algorithm that was developed using a machine learning approach, the individual hemoglobin and iron content of pRBCs can be predicted with a mean accuracy of 97.45 %, 93.37% for the 95-CI and 91.33% accuracy for the 99.7-CI (Figure 5D). Moreover, the inventors identified relevant factors for hemoglobin and iron content in red blood cell units such as unit volume, hemoglobin concentration in blood sample from the fingertip taken prior to donation, sex of donor, separated plasma volume during production, and the blood bag system type, and/or the month of donation. As the inventors have validated the inventor's approach with red blood cell units produced at multiple sites the here presented concept could be applied to any blood bank worldwide with a sufficient amount of local training and validation data.

Regarding the problem of precise Hb and iron content prediction in individual pRBC units, the inventors hypothesized that a multi-feature approach based on the parameters being routinely recorded during blood donation and pRBC production could outperform previous attempts. The inventors therefore tested if supervised ML approaches could provide reliable automatized predictions of Hb and iron content in pRBC units. Since the functional form of the relationship between Hb and iron content and independent parameters was completely unknown, they started with an unbiased approach and thoroughly tested various different ML methods. Specifically, the inventors trained eight different ML algorithms on donation and quality control data of 6,058 pRBC units that were collected between May 2018 and May 2020 and manufactured at five different production sites. Performance of the finally selected model was assessed in a second, independent data set of 2,637 pRBCs of the subsequent year. The inventors verified the hypothesis that a supervised ML approach can deliver a non-invasive, automated method to predict the Hb and iron content in individual pRBC units with high precision. The inventor's concept allows for the first time the reliable declaration of hemoglobin and iron content of individual red blood cell units. Thus, for the first time, red blood cells can be dosed per their pharmaceutically active agent, and the iron uptake in chronically transfused patients can be reliably monitored, which enables clinicians to better control the life-limiting side effects and to optimize the costly iron chelation medication. Eventually, blood banks can, together with clinicians, adjust their transfusion regiments and are enabled to conduct true dose-efficacy studies in various clinical settings hereby promoting patient blood management to the next level.

The present invention will now be described further in the following examples with reference to the accompanying Figures, nevertheless, without being limited thereto, the invention being defined by the appended claims.

Figure 1 shows the distribution of measured Hb and iron content in the training data set (n=6,058). A: Distribution of measured Hb content in 6,058 units. B: Distribution of calculated heme iron content in 6,058 units.

Figure 2 shows the identification of most suitable ML algorithm using all available features (n=6,058). A: Performances of different ML algorithms using all features. MLR = Multiple Linear Regression; RANSAC = Random Sample Consensus; KNN = k-nearest neighbor, DecTree = Decision Tree, SVM = Support Vector Machine; LGBMR = lightGBM Regressor; RF = Random Forest; NN = Neural network. Upper and lower box borders indicate interquartile range (25. - 75. percentile); gray triangles indicate means, light gray lines indicate medians; error bars: 0.3.-99.7. percentile. B: Coefficients for each feature in MLR. C: Relative feature importance in % in MLR.

Figure 3 shows the identification of required minimum number of features in MLR. A: Performances of MLR algorithm in training dataset (n=6,058). 3 = unit volume + sex + Hb fingertip; Month = Month of production; Bag = type of blood bag system. B: Performances of MLR algorithm in validation dataset (n=2,637). C: Scatter plot of prediction residuals for MLR training data set with all features and only 4 features (feature _reduction: unit volume, Hb fingertip, sex and type of blood bag system). Paired t-test; ***p<0.001; n = 6,058. D: Scatter plot of prediction residuals for MLR in validation data set with all features and only 4 features (n=2,637).

Figure 4 shows the identification of most suitable ML algorithm using only 4 features (n=6,058). A: Performances of different ML algorithms using unit volume, Hb fingertip, sex and used type of blood bag system. MLR = Multiple Linear Regression; RANSAC = Random Sample Consensus; KNN = k-nearest neighbor, DecTree =Decision Tree, SVM = Support Vector Machine; LGBMR = lightGBM Regressor; RF = Random Forest; NN = Neural network. Upper and lower box borders indicate interquartile range (25. - 75. percentile); gray triangles indicate means, light gray lines indicate medians; error bars: 0.3.-99.7. percentile. B: Coefficients for each feature in MLR. C: Relative feature importance in % in MLR.

Figure 5 shows the prediction of Hb and iron content in pRBCs using MLR and 4 features. A: Histogram of Hb prediction residuals. B: Histogram of relative residuals to unit Hb in percent for each pRBC unit. C: Histogram of Fe prediction residuals. D: Histogram of relative residuals to unit Fe in percent for each pRBC unit. Dark gray lines indicate the limits of the 68.3% confidence interval ($\mu+\sigma$). Gray lines indicate the limits of the 95.4% confidence interval ($\mu+2*\sigma$). Light gray lines indicate the limits of the 99.7% confidence interval ($\mu+3*\sigma$). n=2,637.

Figure 6 shows the comparison of prediction calculated as proposed in Arslan et al., Agnihotri et al. and the inventors (Epah et al.) to the true Hb content per unit (g). Scatter plot of prediction results to the measured Hb content in g/unit (red) using the Arslan et al. (purple), Agnihotri et al. (green) and the inventors (blue) formulas (n=2,637).

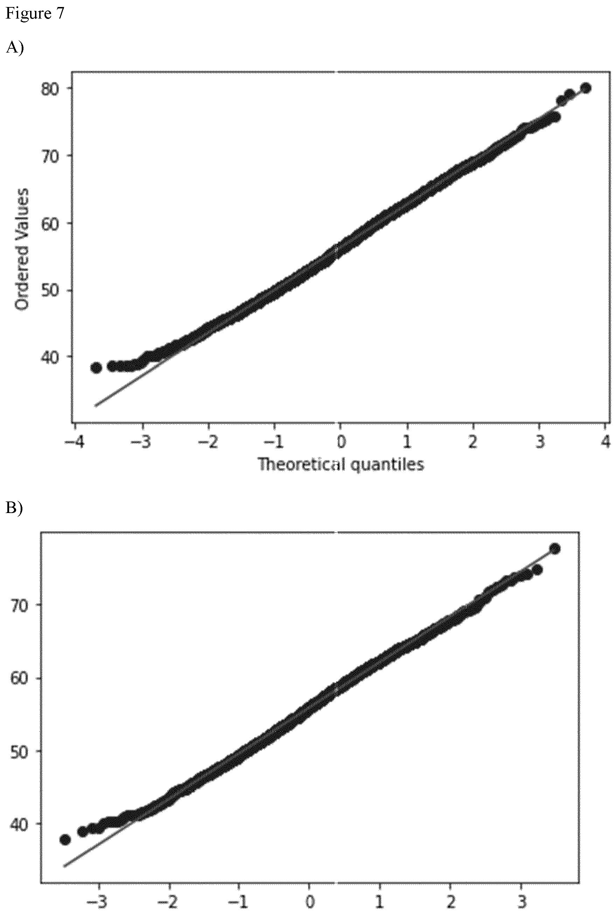

Figure 7 shows that both training and validation cohorts are normally distributed. Normal Q-Q plots comparing data of the target value (total Hb content in g/unit) plotted on the vertical axis to a standard normal population on the horizontal axis. In both training data set (n=6,058) (A) and validation data set (n=2,637) (B) the plotted data match complete linearity (red lines) indicating normal distribution.

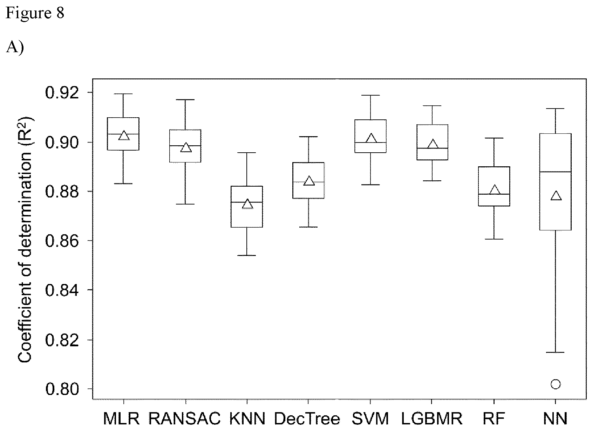

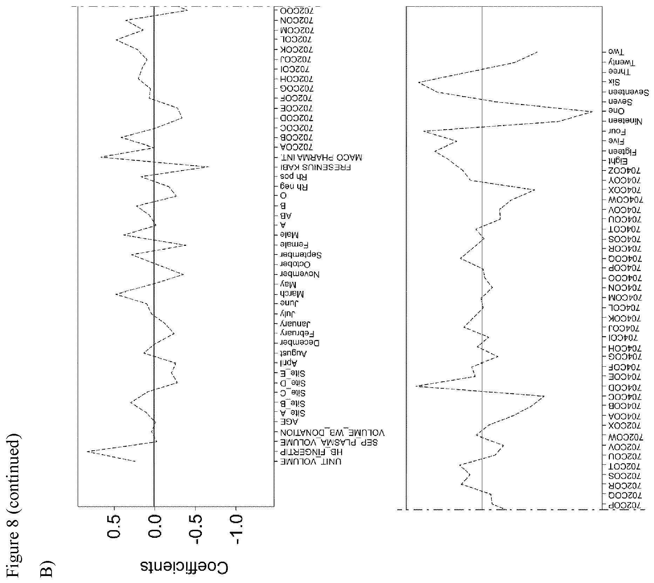

Figure 8 shows that additional features such as production machine and used program did not increase prediction performance (n=6,058). A: Performances of different ML algorithms using additional features. MLR = Multiple Linear Regression; RANSAC = Random Sample Consensus; KNN = k-nearest neighbor, DecTree =Decision Tree, SVM = Support Vector Machine; LGBMR = lightGBM Regressor; RF = Random Forest; NN = Neural network. Upper and lower box borders indicate interquartile range (25. - 75. percentile); Green triangles indicate means, orange lines indicate medians; error bars: 99.7% CI. B: Coefficients for each feature in MLR

Figure 9 shows a navigation graph of a proposed concept for a mobile application according to the present invention. A: Start view, B: Log in view, C: Registration view, D: Patient view, E: Physician view, F: Graphical monitoring of iron levels, and G: Possible gamification option for pediatric patients.

Table 1

| Description of training and validation data sets | | | | | | | | |
|---|---|---|---|---|---|---|---|---|
| *Training Data* | n | mean | SD | min | 25% | 50% | 75% | max |
| Unit volume (mL) | 6,058 | 293.19 | 18.95 | 230 | 279.25 | 293 | 307 | 369 |

(continued)

| Description of training and validation data sets | | | | | | | | |
|---|---|---|---|---|---|---|---|---|
| *Training Data* | n | mean | SD | min | 25% | 50% | 75% | max |
| Unit Hb (g/unit) | 6,058 | 56.21 | 6.35 | 38.5 | 51.6 | 56.1 | 60.8 | 79.9 |
| Fingertip Hb (g/dL) | 6,058 | 14.95 | 1.29 | 11.4 | 14 | 14.9 | 15.9 | 18.9 |
| Separated plasma volume (mL) | 6,058 | 314.72 | 23.24 | 225 | 299 | 315 | 331 | 672 |
| Whole blood donation volume (mL) | 6,058 | 499.71 | 4.52 | 450 | 500 | 500 | 501 | 514 |
| Donor age | 6,058 | 44.04 | 15.04 | 18.00 | 30.39 | 46.17 | 55.89 | 72.80 |
| *Validation Data* | n | mean | SD | min | 25% | 50% | 75% | max |
| Unit volume (mL) | 2,637 | 291.69 | 18.41 | 215.5 | 278.5 | 291.7 | 304.6 | 349.8 |
| Unit Hb (g/unit) | 2,637 | 55.80 | 6.24 | 38 | 51.3 | 55.8 | 60.3 | 77.7 |
| Fingertip Hb (g/dL) | 2,637 | 14.97 | 1.31 | 11.9 | 14 | 14.9 | 15.9 | 18.5 |
| Separated plasma volume (mL) | 2,637 | 313.49 | 22.62 | 211 | 298 | 314 | 330 | 378 |
| Whole blood donation volume (mL) | 2,637 | 499.73 | 4.90 | 450 | 500 | 500 | 501 | 528 |
| Donor age | 2,637 | 44.53 | 15.07 | 18.01 | 30.86 | 47.26 | 56.37 | 72.97 |

| *Training Data* | | *Validation Data* | |
|---|---|---|---|
| *Production Site* | n | *Production Site* | n |
| Site_A | 1,036 | Site_A | 227 |
| Site_B | 1,043 | Site_B | 158 |
| Site_C | 1,027 | Site_C | 808 |
| Site_D | 2,158 | Site_D | 864 |
| Site_E | 794 | Site_E | 580 |
| *Month* | | *Month* | |
| April | 386 | April | 233 |
| August | 435 | August | 186 |
| December | 492 | December | 118 |
| February | 658 | February | 260 |
| January | 923 | January | 88 |
| July | 424 | July | 212 |
| June | 276 | June | 71 |
| March | 731 | March | 300 |
| May | 484 | May | 186 |
| November | 496 | November | 341 |
| October | 388 | October | 252 |
| September | 365 | September | 390 |
| *Sex* | | *Sex* | |
| Female | 2,546 | Female | 1,191 |
| Male | 3,512 | Male | 1,446 |
| *Blood Group* | | *Blood Group* | |
| A | 424 | A | 64 |

(continued)

| Blood Group | | | Blood Group | |
|---|---|---|---|---|
| AB | 1,754 | | AB | 870 |
| B | 3,831 | | B | 1,683 |
| O | 49 | | O | 19 |
| Rhesus | | | Rhesus | |
| Rh neg | 28 | | Rh neg | 9 |
| Rh pos | 6,030 | | Rh pos | 2,628 |
| Blood bag system | | | Blood bag system | |
| CQ42271 FRESENIUS KABI DEUTSCHLAND GMBH | 4,773 | | CQ42271 FRESENIUS KABI DEUTSCHLAND GMBH | 2,279 |
| LQT7248LC MACO PHARMA INT. GMBH | 1,285 | | LQT7248LC MACO PHARMA INT. GMBH | 358 |
| | | | Machine | |
| Machine | | | 702COA | 6 |
| 702COA | 84 | | 702COB | 10 |
| 702COB | 75 | | 702COC | 6 |
| 702COC | 77 | | 702COD | 7 |
| 702COD | 69 | | 702COE | 8 |
| 702COE | 82 | | 702COF | 12 |
| 702COF | 73 | | 702COG | 6 |
| 702COG | 102 | | 702COH | 10 |
| 702COH | 86 | | 702COI | 11 |
| 702COI | 80 | | 702COJ | 14 |
| 702COJ | 86 | | 702COK | 12 |
| 702COK | 83 | | 702COL | 11 |
| 702COL | 81 | | 702COM | 15 |
| 702COM | 82 | | 702CON | 5 |
| 702CON | 88 | | 702COO | 12 |
| 702COO | 73 | | 702COP | 7 |
| 702COP | 73 | | 702COQ | 8 |
| 702COQ | 74 | | 702COR | 12 |
| 702COR | 80 | | 702COS | 2 |
| 702COS | 68 | | 702COT | 8 |
| 702COT | 83 | | 702COU | 10 |
| 702COU | 86 | | 702COV | 12 |
| 702COV | 93 | | 702COW | 16 |
| 702COW | 71 | | 702COX | 14 |
| 702COX | 73 | | 704COE | 167 |
| 704COA | 6 | | 704COF | 149 |
| 704COB | 5 | | 704COG | 160 |

(continued)

| Blood bag system | | Blood bag system | |
|---|---|---|---|
| 704COC | 6 | 704COH | 154 |
| 704COD | 7 | 704COI | 159 |
| 704COE | 268 | 704COJ | 150 |
| 704COF | 143 | 704COK | 142 |
| 704COG | 213 | 704COL | 152 |
| 704COH | 243 | 704COM | 132 |
| 704COI | 184 | 704CON | 146 |
| 704COJ | 246 | 704COO | 101 |
| 704COK | 225 | 704COP | 91 |
| 704COL | 262 | 704COQ | 82 |
| 704COM | 230 | 704COR | 79 |
| 704CON | 223 | 704COS | 70 |
| 704COO | 181 | 704COT | 150 |
| 704COP | 224 | 704COU | 160 |
| 704COQ | 182 | 704COV | 158 |
| 704COR | 180 | Program | |
| 704COS | 211 | Eight | 63 |
| 704COT | 275 | Five | 5 |
| 704COU | 197 | Four | 808 |
| 704COV | 260 | Nineteen | 243 |
| 704COW | 48 | Six | 166 |
| 704COX | 13 | Three | 1,242 |
| 704COY | 51 | Twenty | 110 |
| 704COZ | 53 | | |
| Program | | | |
| Eight | 504 | | |
| Fifteen | 41 | | |
| Five | 450 | | |
| Four | 1,361 | | |
| Nineteen | 367 | | |
| One | 62 | | |
| Seven | 117 | | |
| Seventeen | 3 | | |
| Six | 815 | | |
| Three | 1,997 | | |
| Twenty | 307 | | |
| Two | 34 | | |

Table 2

| | Arslan et al. | Agnihotri et al. | MLR with 4 Features | MLR with 3 Features |
|---|---|---|---|---|
| | | | | |

Model performance metrics for total Hb content predictions in pRBC units as proposed by Arslan et al.,[50] Agnihotri et al.[9] and Epah et al., assessed on the second, independent data set (n=2,637)

| | Arslan et al. | Agnihotri et al. | MLR with 4 Features | MLR with 3 Features |
|---|---|---|---|---|
| Mean of predicted Hb (g/unit) | 74.80 | 69.56 | 55.75 | 55.82 |
| Mean prediction error (g/unit) | -19.0 | -13.76 | 0.05 | -0.02 |
| Mean absolute prediction error (g/unit) | 19.0 | 13.77 | 1.41 | 1.43 |
| Standard deviation of absolute prediction error (g/unit) | 4.22 | 4.03 | 1.13 | 1.13 |
| Mean absolute prediction error (%) | 34.71 | 25.28 | 2.54 | 2.58 |
| Standard deviation of absolute prediction error (%) | 9.45 | 8.75 | 2.04 | 2.07 |
| Mean squared error | 378.71 | 205.78 | 3.26 | 3.32 |
| Standard deviation of mean squared error | 159.90 | 111.47 | 5.41 | 5.44 |
| Adjusted coefficient of determination ($R^2$) | -8.72 (recalibrated[43]: 0.6147) | -4.28 (recalibrated[43]: 0.6148) | 0.92 | 0.91 |

Table 3

| | Mean transfusion rate: 4.1 pRBC units per month over 12 months ($\sum$=49 pRBCs per year) | Required amount of Deferasirox 20mg/kg bw/d Mean net excretion = 0.329 mg Fe/kg bw/d | | Required amount of Deferasirox 40mg/kg bw/d Mean net excretion = 0.445 mg Fe/kg bw/d | |
|---|---|---|---|---|---|

Modelling of iron load and Deferasirox doses and costs based on List et al. [49]

| | Mean transfusion rate: 4.1 pRBC units per month over 12 months ($\sum$=49 pRBCs per year) | Required amount of Deferasirox 20mg/kg bw/d Mean net excretion = 0.329 mg Fe/kg bw/d | | Required amount of Deferasirox 40mg/kg bw/d Mean net excretion = 0.445 mg Fe/kg bw/d | |
|---|---|---|---|---|---|
| Minimum iron content per pRBC unit (133.42 mg) | 17.91 mg Fe/day | 1,088.8 mg /day | 387.61 USD /day | 1,609.9 mg /day | 573.12 USD / day |
| Average iron content per pRBC unit (194.79 mg) | 26.15 mg Fe/day | 1,589.7 mg /day | 565.93 USD /day | 2,350.6 mg /day | 836.6 USD /day |
| Maximum iron content per pRBC unit (276.89 mg) | 37.17 mg Fe/day | 2,259.6 mg /day | 804.42 USD /day | 3,341.1 mg /day | 1,189.43 USD /day |

EXAMPLES

Machine learning allows to predict the highly variable hemoglobin and iron content of individual red blood cell units.

**Material and Methods**

**Production of pRBCs**

Units were produced from whole blood donations varying between 450 to 515 mL collected from male and female European citizens ranging in age from 18 to 73 years, with pre-donation fingertip Hb-levels from 12.5 g/dL for women and 13.5 g/dL for men, up to 16.5 g/dL for women and 18.5 g/dL for men. The whole blood donations were collected with the CompoFlow® blood bag systemCQ42271 by Fresenius Kabi Deutschland GmbH, Bad Homburg von der Höhe, Germany or with the blood bag system LQT7248LC of Maco Pharma GmbH, Langen, Germany and processed with the CompoMat G5 Plus by Fresenius Kabi.

**Hardware**

All operations were performed on a Microsoft Surface Pro 7 with 8GB RAM, Intel® Core™ is (4x 1.10 GHz).

**Software**

For ML and data visualization tasks the open-source ecosystem and data science tool kit Anaconda and its application Jupiter Notebook 6.0.3, Python Version 3.8 and Google Colaboratory was used. Applied programming libraries were Pandas,[19] Numpy,[20] Matplotlib,[21] Scikit learn,[21] Seaborn,[22] Scipy,[23] and TensorFlow[24]/Keras 2.3.1. To use Keras modules within the Scikit learn framework the wrapper scikeras[25] was used. The dNNs were visualized with the extension Graphviz 2.38.[26] For statistical analysis the software Graph Pad Prism 5 was used as well. Super Learner (SL)[27, 28] was applied using the open source MIT tool ML-Ensembl.[29]

**Data Collection**

As per regulatory obligation one percent of the monthly pRBC production is tested for quality control. To obtain representative samples from these pRBC units for analysis they need to be opened, *i.e.* destroyed. The use of data for scientific purposes was approved by the Institutional Review Board of the University Hospital Frankfurt, Germany (Approval# 329/10). The cohorts are described in **Table 1.** To create a training data set the inventors collected manufacturing and quality control data from randomly picked 6,058 pRBCs produced at five different production sites by the German Red Cross Blood Donor Service Baden-Württemberg Hessen in the period of May 2018 to May 2020 **(Table 1).** In detail, the inventors used the inventor's in-house blood banking system (Edgeblood) to extract for each pRBC the following parameters: total Hb content in gram, hematocrit (Hct) in percent, total unit volume in mL, extracted plasma volume in mL, production site, production date, donor age, donor sex, donor Hb in g/dL obtained from obligatory pre-collection fingertip testing, the volume of the whole blood collection in mL, used type of blood bag system, production machine and program **(Table 1).** The inventors repeated this process for the validation data cohort, comprised of 2,637 units, for the period of July 2020 to May 2021 **(Table 1).** The target value total Hb content in grams per unit followed normal distribution in both data sets **(Figure 7).** Features such as production machine and production program did not increase prediction performance **(Figure 8)** but decreased readability of the figures. For the sake of clarity, the inventors therefore excluded them from further analyses.

**Feature engineering and selection**

Since it is not possible to perform calculations with string variables, the inventors transformed categorical variables such as sex and month of production into integer variables using One Hot Coding.[30]To identify the best combination of features the inventors excluded one feature after another and re-assessed the performance.

**Training and evaluation of ML algorithms**

Common ML algorithms such as multiple linear regression (MLR), random sample consensus (RANSAC) algorithm,[31] support vector machine (SVM),[32] Random Forest (RF),[33] k-Nearest Neighbor (KNN),[34] Decision Tree Regressor (DecTree), lightGBM Regressor (lgbmR)[35] and NN were evaluated to determine the best performing ML algorithm using repeated internal k-fold cross validation (k=10) in the Scitkit learn library for three times (n=3). Hyper-parameter tuning for KNN (n_neighbors, weights, algorithm, leaf_size), SVM (C, epsilon, gamma, kernel), DecTree (min_samples_split, min_samples _leaf, splitter), RF (max_features, n_estimators) and NN (activation, optimizer, number of neurons, size of layers, batch size, epochs, optimizer, kernel initializer, dropout rate) were adjusted using Grid-SearchCV. Comparisons of different ML algorithms and selected feature combinations were performed by means of the coefficient of determination ($R^2$): If $\widehat{y_i}$ is the predicted value of the i-th sample $y_i$ and is the corresponding true value, the estimated $R^2$ is defined as:

$$R^2(y, \hat{y}) = 1 - \frac{\sum_{i=1}^{n}(y_i - \hat{y}_i)^2}{\sum_{i=1}^{n}(y_i - \overline{y})^2} \qquad (1)$$

In addition, the prediction results were evaluated calculating mean and standard deviation of absolute and squared error and the absolute error in percent to the actual Hb content in g per unit. For the best performing ML model in terms of highest median $R^2$ on the first data set - in the inventor's case MLR - feature selection was conducted subsequently. Here, feature ranking with recursive feature elimination and cross-validated selection of the best number of features (RFECV) from the Scikit learn library was applied using default settings, a hyper-parameter tuned DecTree Regressor (see above) as estimator and 50-times repeated nested ten-fold cross-validation. Model performance (adjusted $R^2$) of MLR with the best combination of all, three or four features was then evaluated using 50-times repeated ten-fold cross-validation. The two models with the best trade-off between performance and complexity - MLR with 3 and 4 features, respectively - were retrained on the entire first data set (n=6,058) and subsequently evaluated on the second, independent data set (n=2,637) for an unbiased assessment of model performance.

**Calculating pRBC units heme iron content**

Given a mean molecular weight of the Hb tetramer of 64.458 g/mmol[36] and the fact that one Hb tetramer contains four iron atoms with a molar mass of 55.845 g/mol applying the common rules of chemical stoichiometry the inventors utilized the following formula:

$$heme\ iron\ (mg/unit) = \frac{x\ g\ \frac{Hb}{unit} * 4\ Fe * 55.845\ g/mol}{64.458\ g/mmol} \qquad (2)$$

$x$ = predicted or measured Hb content in *g Hb/unit*

**Pilot study for Hb content calculation in pRBC units**

The inventors randomly picked pRBCs (n = 9) independent from the first as well as the second cohort. For these samples, the inventors compared the measured Hb content and the calculated Hb content based on the formulars of Arslan *et al.* [15] and Agnihotri *et al.* [9]. After weighing and gently shaking on a rocker, samples from the pRBC units were taken in S-Monovettes® with potassium EDTA, 2.7 mL (Sarstedt AG & Co. KG, Nümbrecht, Germany). Hb concentration in g dL$^{-1}$ and Hct in % were determined by two repeated measurements with the Sysmex XN-350 System (Sysmex Deutschland GmbH, Norderstedt, Germany). Unit volume was calculated dividing the measured weight by the relative density of blood[42] adjusted to the measured Hct in %. Total Hb content in the pRBC units was calculated by multiplying the measured Hb concentration with the respective unit volume.

**Calculating the Hb content in pRBC units as proposed by Arslan *et al.* [15] and Atilla *et al.* [18]**

$$Predicted\ total\ Hb\ in\ unit\ (g) = Whole\ blood\ donation\ (L) * Fingertip\ Hb\ \left(\frac{g}{L}\right) \qquad (3)$$

**Calculating the Hb content in pRBC units as proposed by Agnihotri *et al.* [9]**

$$Predicted\ total\ Hb\ in\ unit\ (g)$$
$$= Whole\ blood\ donation\ (L) * Fingertip\ Hb\ \left(\frac{g}{L}\right) - 0.035\ (L) * Fingertip\ Hb\ \left(\frac{g}{L}\right) \qquad (4)$$

**Results**

**Hemoglobin and iron content is highly variable in pRBC units.**

When the inventors analyzed the first data set of 6,058 pRBC units the inventors found a great variability of the Hb content ranging from 38.5 g to 79.9 g per unit with a mean of 56.21 g Hb. They discovered that, only 60% of all tested pRBC units contained 56.21g $\pm$ 10% of the mean (5.62 g) Hb, whereas 32% deviated greater than 10% of the mean from the mean and 8% even greater than 20%. **(Figure 1A).**

As shown below in (9) heme iron represents total iron within a pRBC unit and is determined by the Hb content. Therefore, the inventors exactly calculated heme iron based on the respective Hb levels. According to the Hb distribution 60% of the pRBC units contained 188.93 $\pm$ 18.88 mg iron, whereas 16% contained 160.51 $\pm$ 9.44 mg iron, 16% contained 217.17 $\pm$ 9.44 mg iron, 3% contained 141.63 $\pm$ 9.44 mg iron, and 3% contained 236.05 $\pm$ 9.44 mg iron **(Figure 1B).**

**Previous Hb content prediction approaches substantially overestimate Hb content in pRBC units.**

In a pilot study comprising 9 pRBC units, the inventors used the previous approaches developed by Arslan *et al.*[15] and Agnihotri *et al.* [9] to estimate the Hb content. Of note, both approaches substantially overestimated the measured (real) Hb content with mean prediction errors of 19.55 g Hb/unit (30.8%) and 14.35 g Hb/unit (26.4%) **(Figure 6).**

**Multiple linear regression, SVM and LGBMR are best performing machine learning models for Hb and iron prediction in pRBC units.**

As the previously developed approaches showed poor accuracy in the inventor's pilot study, the inventors sought to develop the inventor's own model to predict Hb and iron content of any given pRBC unit without compromising the integrity of these products. Therefore, the inventors considered all (thirteen) features that were routinely collected during the blood donation, pRBC production and quality control, and evaluated eight different ML methods on the first data. Specifically, the inventors found that MLR, RANSAC, SVM and LGBMR slightly outperformed DecTree, KNN, RF and NN with respect to median $R^2$ on the first data set, with MLR performing the best (median $R^2$= 0.903; 95% confidence interval = 0.885 - 0.921) **(Figure 4A).**

**Recursive feature elimination selects unit volume, donor Hb obtained fingertip testing and donor sex as the best feature combination in MLR.**

Out of the best performing ML methods the inventors selected the MLR for its simplicity and easy applicability and used cross-validation based feature section (RFECV) on the first data set (n=6,058) to identify the optimal number of features. This number turned out to be three with the best combination being unit volume, donor Hb in g dL$^{-1}$ obtained by fingertip testing, and donor sex. Nevertheless, the inventors observed that the model with four features performed almost as good with a slightly increased mean and median cross-validated $R^2$ and slightly decreased mean and median cross-validated MSE. Here, the blood bag system was additionally part of the best combination. Interestingly, in each of the 50 cross-validation repeats, the same features for the three as well as for the four-feature model were selected. Both sets of features reached almost an as high adjusted $R^2$ as the MLR with all features (mean/median ~0.9), when applying the models on the entire first data set. Since the inventors supposed that the feature blood bag system may be relevant in the production process, the inventors decided to consider both MLR with three and four features for an unbiased assessment of model performance in the inventor's second, independent data set (n=2,637).

Considering the above described iterative process, the inventors propose the two following formulas for Hb and iron prediction in pRBC units:

**Formula for MLR with three features:**

$$Predicted\ total\ Hb\ in\ unit\ (g) =$$

$$\beta0 + \beta1 * Unit\ volume\ (mL) + \beta2 * Hb\ Fingertip\left(\frac{g}{dL}\right) + \beta3 * xi3 + \beta4 * xi4 \tag{5}$$

$$\beta0= (-32.771);\ \beta1= 0.254;\ \beta2= 0.964;$$

$$\beta 3 = (-0.426); \ \beta 4 = 0.426$$

xi3= 1 if female, else 0;
xi4= 1 if male, else 0,

**Formula for MLR with four features:**

$$Predicted \ total \ Hb \ in \ unit \ (g) =$$

$$\beta 0 + \beta 1 * Unit \ volume \ (mL) + \ \beta 2 * Hb \ Fingertip \left(\frac{g}{dL}\right) + \ \beta 3 * xi3 + \qquad (6)$$

$$\beta 4 * xi4 + \beta 5 * xi5 \ + \ \beta 6 * xi6$$

$$\beta 0 = (-33.\ 972); \ \beta 1 = 0.263; \ \beta 2 = 0.893;$$

$$\beta 3 = (-0.386); \ \beta 4 = 0.386; \ \beta 5 = (-0.371) \ ; \ \beta 6 = 0.371$$

xi3= 1 if female, else 0;
xi4= 1 if male, else 0,
xi5 = 1 if blood bag system CQ42271 FRESENIUS KABI DEUTSCHLAND GMBH else 0.;
xi6 = 1 if blood bag system LQT7248LC MACO PHARMA INT. GMBH else 0;

**Equation to convert predicted Hb content/pRBC unit to iron content/pRBC unit**

$$Predicted \ total \ iron \ in \ unit \ (mg) =$$

$$\left(\frac{x * 4 \ Fe * 55.845 \ g/mol}{64.458 \ g/mmol}\right) \qquad (7)$$

x = predicted or measured Hb content in *g Hb/unit*

This prediction of total Hb and heme iron content allows also to estimate Hb and heme iron concentration per milliliter and, thus, to declare these dosing parameters on the pRBC unit label.

**Unbiased assessment of model fit in a second, independent data set confirms excellent performance of MLR models.**

For unbiased assessment of the inventor's final MLR models with three or four features (see formulas (5) and (6)), the inventors used a second, independent data set of 2,637 pRBCs randomly collected during June 2020 and May 2021 **(Table 1).** Both MLR models showed similar performances on this second data set **(Table 2).**

In detail, the MLR model with three features reached an adjusted $R^2$ of 0.91, and with four features of 0.92. This is in the range of observed $R^2$ values of the nested CV in the first data set with a mean adjusted $R^2$ of 0.896 for three features and 0.898 for four features with standard deviations for both of 0.01. The mean prediction error in the second independent cohort for the inventor's MLR model with three features was -0.02 g Hb/ -0.06 mg iron, and with four features 0.05 g Hb / 0.17 mg iron. The limits of the 95% CI were $\pm 3.57$ g Hb/ 12.01 mg iron for MLR with three features and $\pm 3.54$ g Hb / 11.89 mg iron with four features **(Figure 5A, B).** The mean absolute prediction error in % of the measured Hb/iron content was 2.58% for the MLR with three features, 2.55% for the MLR with four features, and the upper limit of the corresponding 95% CI was 6.55% for both **(Figure 5C, D).**

**MLR models with both three and four features achieve unprecedented accuracy and precision for predicting Hb and iron content in pRBC units.**

Finally, the inventors compared the inventor's both ML-derived formulas with the previous approaches for Hb prediction on the inventor's second, independent data set (n=2, 637). MLR models with three as well as four features predicted Hb content highly accurate with a mean absolute prediction error of 2.58% and 2.54%, respectively, of the Hb content of a given unit, whereas both Arslan et al.[15] as well Agnihotri et al. [9] overestimated the measured Hb content significantly, with mean absolute prediction errors of 18.997 g Hb/unit (34.71%) and 13.767 g Hb/unit (25.28%) **(Figure 6; Table 2)**. In addition, the previously published models featured a clearly increased variability of predicted Hb contents compared to the inventor's MLR models with three or four features. The low $R^2$ values of -8.72 (recalibrated[43]: 0.6147) for Arslan et al. and -4.28 (recalibrated[43]: 0.6148) for Agnihotri et al. obviously illustrate their weak performance, especially in light of $R^2$ values above 0.9 for the inventor's models **(Table 2)**. Agreement between measured and predicted Hb content was also graphically assessed via Bland-Altman plots[44]. With a mean prediction error of -0.02 g Hb/ -0.06 mg iron for MLR with three features and 0.05 g Hb / 0.17 mg iron for four features the estimated bias is negligible and 95% of the absolute prediction errors were below 3.59 g Hb/ 12.08 mg iron.

**Significant dose and cost savings can be achieved by dosing chelation therapy based on accurate predictions of iron content in pRBC units.**

Demonstrating a use case of a high prediction accuracy for Hb and iron content in pRBC units the inventors modeled the iron load, corresponding Deferasirox doses and costs based on List et al. (58). Based on conservative estimations of U.S. drug prices and the minimum, average and maximum iron content per pRBC unit, dose and cost savings of up to 50%, or 597,77$ per day can be achieved when applying the here presented algorithm **(Table 3)**.

**References**

1. World Health Organization (WHO). Global Forum for Blood Safety: Patient Blood Management; https://www.who.int/bloodsafety/events/gtbs_01_pbm/en/. 2011.

2. Müller,M.M., Geisen,C., Zacharowski,K., Tonn,T., & Seifried,E. Transfusion of Packed Red Cells. Dtsch Arztebl Int 112, 507-518 (2015).

3. Christou,G. et al. Optimal transfusion practices after allogeneic hematopoietic cell transplantation: a systematic scoping review of evidence from randomized controlled trials. Transfusion 56, 2607-2614 (2016).

4. Shander,A., Cappellini,M.D., & Goodnough,L.T. Iron overload and toxicity: the hidden risk of multiple blood transfusions. Vox Sang. 97, 185-197 (2009).

5. Neukirchen,J. et al. Improved survival in MDS patients receiving iron chelation therapy - a matched pair analysis of 188 patients from the Dusseldorf MDS registry. Leuk. Res. 36, 1067-1070 (2012).

6. Remacha,A. et al. Guidelines on haemovigilance of post-transfusional iron overload. Blood Transfus. 11, 128-139 (2013).

7. Haghpanah,S., Zarei,T., Zahedi,Z., & Karimi,M. Compliance and satisfaction with deferasirox (Exjade(R)) compared with deferoxamine in patients with transfusion-dependent beta-thalassemia. Hematology. 19, 187-191 (2014).

8. Zurlo,M.G. et al. Survival and causes of death in thalassaemia major. Lancet 2, 27-30 (1989).

9. Agnihotri,N., Pal,L., Thakur,M., & Kumar,P. The need to label red blood cell units with their haemoglobin content: a single centre study on haemoglobin variations due to donor-related factors. Blood Transfus. 12, 520-526 (2014).

10. Porter,J. & Viprakasit,V. Iron Overload and Chelation in Guidelines for the Management of Transfusion Dependent Thalassaemia (TDT) [Internet]. 3rd edition (eds. Cappellini,M.D., Cohen,A. & Porter,J.) (Thalassaemia International Federation, 2014).

11. Lau,W. Neonatal and Pediatric Transfusion. [Chapter 13]. 2017. Canadian Blood Services. Clinical Guide to Transfusion.

12. Ness,P.M. & Kruskall,M.S. Principles of Red Blood Cell Transfusion in Hematology: Basic Principles and Practice (ed. Hoffman,K.) 2005).

13. Ali,A., Auvinen,M.K., & Rautonen,J. The aging population poses a global challenge for blood services. Transfusion 50, 584-588 (2010).

14. Greinacher,A., Fendrich,K., & Hoffmann,W. Demographic Changes: The Impact for Safe Blood Supply. Transfus. Med. Hemother. 37, 141-148 (2010).

15. Arslan,O. Hemosoft: a new software for blood bank and apheresis management. Transfus. Apher. Sci. 30, 193-196 (2004).

16. Atilla,E., Toprak,S.K., Civriz,B.S., Topcuoglu,P., & Arslan,O. A Randomized Comparison of Hemoglobin Content-Based Versus Standard (Unit-Based) Red Blood Cell Transfusion Policy. Turk. J. Haematol. 34, 244-249 (2017).

17. Esteva,A. et al. Dermatologist-level classification of skin cancer with deep neural networks. Nature 542, 115-118 (2017).

18. Anderson,J.P. et al. Reverse Engineering and Evaluation of Prediction Models for Progression to Type 2 Diabetes: An Application of Machine Learning Using Electronic Health Records. J. Diabetes Sci. Technol. 10, 6-18 (2015).

19. McKinney,W. Data Structures for Statistical Computing in Python. Proceedings of the 9th Python in Science Conference. 9th Python in Science Conference. Proceedings of the 9th Python in Science Conference 445, 51-56. 2010.

20. Harris,C.R. et al. Array programming with NumPy. Nature 585, 357-362 (2020).

21. Hunter,J.D. Matplotlib: A 2D Graphics Environment. Computing in Science & Engineering 9, 90-95 (2007).

22. Waskom,W. et al. mwaskom/seaborn: v0.8.1 (September 2017); https://doi.org/10.5281/zenodo.883859. 2017. doi.org/10.5281/zenodo.883859.

23. Virtanen,P. et al. SciPy 1.0: fundamental algorithms for scientific computing in Python. Nat. Methods 17, 261-272 (2020).

24. Abadi,M. et al. TensorFlow: A system for large-scale machine learning. 12th USENIX Symposium on Operating Systems Design and Implementation (OSDI 16). 265-283. 2016. USENIX Association.

25. scikeras 0.2.1; https://pypi.org/project/scikeras/. 2020.

26. Squarify; https://pypi.org/project/squarify/. 2019.

27. Gansner,E.R. & North,S.C. An open graph visualization system and its applications to software engineering . Software Practice and Experience 30, 1203-1233 (2000).

28. van der Laan,M.J., Polley,E.C., & Hubbard,A.E. Super learner. Stat. Appl. Genet. Mol. Biol. 6, Article25 (2007).

29. Polley,E.C. & van der Laan,M.J. Super Learner In Prediction. Working Paper 266. 2010. U.C. Berkeley Division of Biostatistics Working Paper Series.

30. Flennerhag,S. mlens Documentation; Release 0.1.6.; https://mlens.readthedocs.io/ /downloads/en/0.1.x/pdf/. 2017.

31. Guyon,I. & Elisseeff,A. An introduction to variable and feature selection. Machine Learning 3, 1157-1182 (2003).

32. Breiman,L., Friedman,J., Stone,C.J., & Olshen,R.A. Classification and Regression Trees(Chapman and Hall/CRC, Boca Raton, 1984).

33. Potdar,K., Pardawala,T.S., & Pai,C.D. A Comparative Study of Categorical Variable Encoding Techniques for Neural Network Classifiers. International Journal of Computer Applications 175, 7-9 (2017).

34. Fischler,M.A. & Bolles,R.C. Random sample consensus: a paradigm for model fitting with applications to image analysis and automated cartography. Communications of the ACM 24, 381-395 (1981).

35. Cortes,C. & Vapnik,V. Support-vector networks. Machine Learning 20, 273-297 (1995).

36. Breiman,L. Random Forests. Machine Learning 45, 5-32 (2001).

37. Fix,E. & Hodges,J.L. Discriminatory Analysis, Nonparametric Discrimination: Consistency Properties. Technical Report. 4. 1951. USAF School of Aviation Medicine, Randolph Field, TX.

38. Ke,G. et al. LightGBM: a highly efficient gradient boosting decision tree. 31st International Conference on Neural Information Processing Systems (NIPS'17). Proceedings of the 31st International Conference on Neural Information Processing Systems (NIPS'17) , 3149-3157. 2017. Red Hook, NY, Curran Associates Inc.

39. BRAUNITZER,G. THE MOLECULAR WEIGHT OF HUMAN HAEMOGLOBIN. Bibl. Haematol. 18, 59-60 (1964).

40. Trudnowski,R.J. & Rico,R.C. Specific gravity of blood and plasma at 4 and 37 degrees C. Clin. Chem. 20, 615-616 (1974).

41. Davenport,R. Blood components should be labeled for content. Transfusion 45, 3-4 (2005).

42. Lo,B.D. et al. Impact of body weight on hemoglobin increments in adult red blood cell transfusion. Transfusion(2021).

43. Mueller,M.M. et al. Patient Blood Management: Recommendations From the 2018 Frankfurt Consensus Conference. JAMA 321, 983-997 (2019).

44. Volken,T. et al. Red blood cell use in Switzerland: trends and demographic challenges. Blood Transfus. 16, 73-82 (2018).

45. Bolton-Maggs,P.H.B. & Watt,A. Transfusion errors - can they be eliminated? Br. J. Haematol. 189, 9-20 (2020).

46. Doan,M. et al. Objective assessment of stored blood quality by deep learning. Proc. Natl. Acad. Sci. U. S. A 117, 21381-21390 (2020).

47. Geman,S., Bienenstock,E., & Doursat,R. Neural Networks and the Bias/Variance Dilemma. Neural Computation 4, 1-58 (1992).

48. Anscombe,F.J. Graphs in Statistical Analysis. The American Statistician 27, 17-21 (1973).

49. https://www.drk-haemotherapie.de/ausgaben/ausgabe-32-2019. DRK Hämotherapie . 2019.

50. Guidelines for the Management of Transfusion Dependent Thalassaemia (TDT)(Thalassaemia International Federation,2014).

51. Cazzola,M. et al. A moderate transfusion regimen may reduce iron loading in beta-thalassemia major without producing excessive expansion of erythropoiesis. Transfusion 37, 135-140 (1997).

52. List,A.F. et al. Deferasirox reduces serum ferritin and labile plasma iron in RBC transfusion-dependent patients with myelodysplastic syndrome. J. Clin. Oncol. 30, 2134-2139 (2012).

53. Arslan,O., Toprak,S., Arat,M., & Kayalak,Y. Hb content-based transfusion policy successfully reduces the number of RBC units transfused. Transfusion 44, 485-488 (2004).

**Claims**

1. A computer-implemented method for predicting total Hb (g) in a unit of packed red blood cells (pRBC), comprising

   a) obtaining data for i) the volume (mL) of the unit, ii) the concentration of Hb Fingertip (g/dL), iii) the sex of the donor, and iv) type of blood bag system of the pRBC, and
   b) predicting total Hb (g) in said unit using the following equation

$$\beta0 + Unit\ volume\ (mL)\beta1\ +\ Hb\ Fingertip\ \left(\tfrac{g}{dL}\right)\beta2\ +\ xi3\beta3\ +\ xi4\beta4 + xi5\beta5\ +$$
$$xi6\beta6\,,$$

   wherein

$$\beta0= (-33.\ 97164897288266);\ \beta1= 0.263;\ \beta2= 0.893;$$

$$\beta3= (-0.386);\ \beta4 = 0.386,$$

$$\beta5 = (-0.371);\ \beta6 = 0.371$$

   *xi3= 1 if female, else 0; xi4= 1 if male, else 0, xi5 = 1 if blood bag system CQ42271 FRESENIUS KABI DEUTSCHLAND GMBH else 0; xi6 = 1 if blood bag system LQT7248LC MACO PHARMA INT. GMBH else 0;*

2. The method according to claim 1, further comprising the step of c) assigning the unit of pRBC to a group of pRBCs suitable for transplantation or not, and/or assigning the unit of pRBC as suitable or not for a given patient or group of patients, based on said predicting of total Hb (g).

3. A computer-implemented method for predicting total iron (mg) in a unit of packed red blood cells (pRBC), comprising

   a) obtaining data for i) the volume (mL) of the unit, ii) the concentration of Hb Fingertip (g/dL), iii) the sex of the donor, and iv) used type of blood bag system of the pRBC, and
   b) predicting total iron (mg) in said unit using the following equation

$$\frac{(\beta0 + \beta1 * Unit\ volume\ (mL) +\ \beta2 * Hb\ Fingertip\ \left(\tfrac{g}{dL}\right)\ +\ \beta3 * xi3\ +\ \beta4 * xi4 + \beta5 * xi5\ +\ \beta6 * xi6) * 4\ Fe * 55.845\frac{g}{mol}}{64.458\ \frac{g}{mmol}}$$

   wherein

$$\beta0= (-33.\ 97164897288266);\ \beta1= 0.263;\ \beta2= 0.893;$$

$$\beta3= (-0.386);\ \beta4 = 0.386,$$

$$\beta5 = (-0.371);\ \beta6 = 0.371$$

*xi3= 1 if female, else 0; xi4= 1 if male, else 0, xi5 = 1 if blood bag system CQ42271 FRESENIUS KABI DEUTSCHLAND GMBH else 0; xi6 = 1 if blood bag system LQT7248LC MACO PHARMA INT. GMBH else 0;*

4. The method according to claim 3, further comprising the step of c) assigning the unit of pRBC to a group of pRBCs suitable for transplantation or not, and/or assigning the unit of pRBC as suitable or not for a given patient or group of patients, based on said predicting of total iron (mg).

5. A computer-implemented method for predicting total iron (mg) and total Hb (g) in a unit of packed red blood cells (pRBC), comprising combining the methods according to claim 1 and 3.

6. The method according to claim 5, further comprising the step of c) assigning the unit of pRBC to a group of pRBCs suitable for transfusion or not and/or assigning the unit of pRBC as suitable or not for a given patient or group of patients, based on said predicting of total iron (mg) and/or total Hb (g).

7. The method according to any one of claims 1 to 6, wherein the prediction of Hb has a mean accuracy of 97.45 %, 93.37% for the 95-CI and 91.33% accuracy for the 99.7-CI, that lead to a confidence interval of about 2.54 g, and/or the iron content has a confidence interval of about 8.80 mg in about 70% of red blood cells in the units as included in the prediction.

8. The method according to any one of claims 1 to 7, wherein the data in step a) is obtained by a data recording device, such as, for example, a bar code scanner.

9. The method according to any one of claims 1 to 8, further comprising the step of determining the total iron (mg) and/or total Hb (g) in said unit.

10. The method according to any one of claims 1 to 9, wherein said method is automated, such as, for example, performed by a robot.

11. A data processing apparatus comprising means for carrying out the method of claims 1-10.

12. A computer program comprising instructions which, when the program is executed by a computer, cause the computer to carry out the method of any one of claims 1 to 10.

13. A computer-readable medium having stored thereon the computer program of claim 12.

**Patentansprüche**

1. Computer-implementiertes Verfahren zur Vorhersage von Gesamt Hb (g) in einer Einheit von gepackten roten Blutzellen (pRBC), umfassend

   a) Erhalten von Daten für i) das Volumen (mL) der Einheit, ii) die Konzentration von Hb Fingerspitze (g/dL), iii) das Geschlecht des Spenders und iv) den Typ von Blutbeutelsystem der pRBC, und
   b) Vorhersagen von Gesamt Hb (g) in der Einheit unter der Verwendung der folgenden Gleichung

$$\beta 0 + Volumen\ der\ Einheit\ (mL)\beta 1\ +\ Hb\ Fingerspitze\ \left(\frac{g}{dL}\right)\beta 2\ +\ xi3\beta 3\ +\ xi4\beta 4 + xi5\beta 5\ +\ xi6\beta 6\,,$$

wobei

$$\beta 0= (-33.\ 97164897288266);\ \beta 1= 0.263;\ \beta 2= 0.893;$$

$$\beta 3= (-0.386);\ \beta 4 = 0.386,$$

$$\beta5 = (-0.371); \beta6 = 0.371$$

*xi3= 1 wenn weiblich, anderenfalls 0; xi4= 1 wenn männlich, anderenfalls 0, xi5 = 1 wenn Blutbeutelsystem CQ42271 FRESENIUS KABI DEUTSCHLAND GMBH anderenfalls 0; xi6 = 1 wenn Blutbeutelsystem LQT7248LC MACO PHARMA INT. GMBH anderenfalls 0;*

2. Verfahren nach Anspruch 1, weiter umfassend den Schritt von c) Zuordnen der Einheit von pRBC zu einer Gruppe von pRBCs die für die Transplantation geeignet sind oder nicht, und/oder Zuordnen der Einheit von pRBC als geeignet oder nicht für eine bestimmten Patienten oder Gruppe von Patienten, basierend auf der Vorhersage von Gesamt Hb (g).

3. Computer-implementiertes Verfahren zur Vorhersage von Gesamt Eisen (mg) in einer Einheit von gepackten roten Blutzellen (pRBC), umfassend

a) Erhalten von Daten für i) das Volumen (mL) der Einheit, ii) die Konzentration von Hb Fingerspitze (g/dL), iii) das Geschlecht des Spenders und iv) den Typ von Blutbeutelsystem der pRBC, und
b) Vorhersagen von Gesamt Eisen (mg) in der Einheit unter der Verwendung der folgenden Gleichung

$$\frac{(\beta0 + \beta1 * Volumen\ der\ Einheit\ (mL) + \beta2 * Hb\ Fingerspitze\ \left(\frac{g}{dL}\right) + \beta3 * xi3 + \beta4 * xi4 + \beta5 * xi5 + \beta6 * xi6) * 4\ Fe * 55.845\frac{g}{mol}}{64.458\ \frac{g}{mmol}}$$

wobei

$$\beta0= (-33.97164897288266); \beta1= 0.263; \beta2= 0.893;$$

$$\beta3= (-0.386); \beta4 = 0.386,$$

$$\beta5 = (-0.371); \beta6 = 0.371$$

*xi3= 1 wenn weiblich, anderenfalls 0; xi4= 1 wenn männlich, anderenfalls 0, xi5 = 1 wenn Blutbeutelsystem CQ42271 FRESENIUS KABI DEUTSCHLAND GMBH anderenfalls 0; xi6 = 1 wenn Blutbeutelsystem LQT7248LC MACO PHARMA INT. GMBH anderenfalls 0;*

4. Verfahren nach Anspruch 3, weiter umfassend den Schritt von c) Zuordnen der Einheit von pRBC zu einer Gruppe von pRBCs die für die Transplantation geeignet sind oder nicht, und/oder Zuordnen der Einheit von pRBC als geeignet oder nicht für eine bestimmten Patienten oder Gruppe von Patienten, basierend auf der Vorhersage von Gesamt Eisen (mg).

5. Computer-implementiertes Verfahren zur Vorhersage von Gesamt Eisen (mg) und Gesamt Hb (g) in einer Einheit von gepackten roten Blutzellen (pRBC), umfassend ein Kombinieren der Verfahren nach Anspruch 1 und 3.

6. Verfahren nach Anspruch 5, weiter umfassend den Schritt von c) Zuordnen der Einheit von pRBC zu einer Gruppe von pRBCs die für die Transplantation geeignet sind oder nicht, und/oder Zuordnen der Einheit von pRBC als geeignet oder nicht für eine bestimmten Patienten oder Gruppe von Patienten, basierend auf der Vorhersage von Gesamt Eisen (mg) und/oder Gesamt Hb (g).

7. Verfahren nach einem der Ansprüche 1 bis 6, wobei die Vorhersage von Hb eine mittlere Genauigkeit von 97,45 %, 93,37% für das 95-CI und 91,33% Genauigkeit für das 99,7-CI aufweist, was zu einem Konfidenzintervall von ungefähr 2.54 g führt, und/oder der Eisengehalt ein Konfidenzintervall von ungefähr 8,80 mg in ungefähr 70% der roten Blutzellen in den Einheiten, die in der Vorhersage enthalten sind, aufweist.

8. Verfahren nach einem der Ansprüche 1 bis 7, wobei die Daten in Schritt a) durch ein Datenaufzeichnungsgerät, wie zum Beispiel einen Barcodescanner, erhalten werden.

9. Verfahren nach einem der Ansprüche 1 bis 8, weiter umfassend den Schritt von Bestimmen des Gesamt Eisen (mg) und/oder Gesamt Hb (g) in der Einheit.

10. Verfahren nach einem der Ansprüche 1 bis 9, wobei das Verfahren automatisiert ist, zum Beispiel durch einen Roboter durchgeführt wird.

11. Daten verarbeitende Vorrichtung, umfassend Mittel zur Durchführung des Verfahrens der Ansprüche 1 bis 10.

12. Computerprogramm umfassend Anweisungen, die, wenn das Programm von einem Computer ausgeführt wird, den Computer dazu veranlassen das Verfahren nach einem der Ansprüche 1 bis 10 auszuführen.

13. Computer-lesbares Medium, welches das Computerprogramm nach Anspruch 12 darauf gespeichert aufweist.

**Revendications**

1. Procédé mis en œuvre par ordinateur pour prédire l'hémoglobine totale (g) dans une unité de concentré de globules rouges (CGR), comprenant a) l'obtention de données pour i) le volume (mL) de l'unité, ii) la concentration d'hémoglobine au bout du doigt (g/dL), iii) le sexe du donneur, et iv) le type de système de poche de sang dans le CGR, et

b) la prédiction de l'hémoglobine totale (g) dans ladite unité à l'aide de l'équation suivante

$\beta 0$ + volume de l'unité (mL)$\beta 1$ + hémoglobine au bout du doigt (g/dL)$\beta 2$ + xi3$\beta 3$ + xi4$\beta 4$ + xi5$\beta 5$ + xi6f36,

dans laquelle

$$\beta 0 = (-33, 97164897288266) ; \beta 1 = 0,263 ; \beta 2 = 0,893 ;$$

$$\beta 3 = (-0,386) ; \beta 4 = 0,386,$$

$$\beta 5 = (-0,371) ; \beta 6 = 0,371$$

xi3 = 1 si femme, sinon 0 ; xi4 = 1 si homme, sinon 0 ; xi5 = 1 si système de poche de sang CQ42271 FRESENIUS KABI DEUTSCHLAND GMBH, sinon 0 ; xi6 = 1 si système de poche de sang LQT7248LC MACO PHARMA INT. GMBH, sinon 0.

2. Procédé selon la revendication 1, comprenant en outre l'étape c) consistant à attribuer l'unité de CGR à un groupe de CGRs adapté ou non à la transplantation, et/ou attribuer l'unité de CGR comme étant adaptée ou non à un patient ou à un groupe de patients donné, sur la base de ladite prédiction de l'hémoglobine totale (g).

3. Procédé mis en œuvre par ordinateur pour prédire le fer total (mg) dans une unité de concentré de globules rouges (CGR), comprenant

a) l'obtention de données pour i) le volume (mL) de l'unité, ii) la concentration d'hémoglobine au bout du doigt (g/dL), iii) le sexe du donneur, et iv) le type de système de poche de sang utilisé pour le CGR, et
b) la prédiction de fer total (mg) dans ladite unité à l'aide de l'équation suivante

$[(\beta 0 + \beta 1$ * volume de l'unité (mL) + $\beta 2$ * hémoglobine au bout du doigt (g/dL) + $\beta 3$ * xi3 + $\beta 4$ * xi4 + $\beta 5$ * xi5 + $\beta 6$ * xi6) * 4 Fe * 55,845 (g/mol)] / 64,458 (g/mmol)

dans laquelle

$$\beta 0 = (-33, 97164897288266) ; \beta 1 = 0,263 ; \beta 2 = 0,893 ;$$

$$\beta 3 = (-0,386) ; \beta 4 = 0,386,$$

$$\beta5 = (-0,371) \; ; \; \beta6 = 0,371$$

*xi3 = 1 si femme, sinon 0 ; xi4 = 1 si homme, sinon 0 ; xi5 = 1 si système de poche de sang CQ42271 FRESENIUS KABI DEUTSCHLAND GMBH, sinon 0 ; xi6 = 1 si système de poche de sang LQT7248LC MACO PHARMA INT. GMBH, sinon 0.*

4. Procédé selon la revendication 3, comprenant en outre l'étape c) consistant à attribuer l'unité de CGR à un groupe de CGRs adapté ou non à la transplantation, et/ou attribuer l'unité de CGR comme étant adaptée ou non à un patient ou un groupe de patients donné, sur la base de ladite prédiction du fer total (mg).

5. Procédé mis en œuvre par ordinateur pour prédire le fer total (mg) et l'hémoglobine totale (g) dans une unité de concentré de globules rouges (CGR), comprenant la combinaison des procédés selon les revendications 1 et 3.

6. Procédé selon la revendication 5, comprenant en outre l'étape c) consistant à attribuer l'unité de CGR à un groupe de CGRs adapté ou non à la transfusion et/ou attribuer l'unité de CGR comme étant adaptée ou non à un patient ou un groupe de patients donné, sur la base de ladite prédiction du fer total (mg) et/ou de l'hémoglobine totale (g).

7. Procédé selon l'une quelconque des revendications 1 à 6, dans lequel la prédiction de l'hémoglobine a une précision moyenne de 97,45 %, 93,37 % pour l'IC à 95 % et 91,33 % pour l'IC à 99,7 %, ce qui conduit à un intervalle de confiance d'environ 2,54 g, et/ou la teneur en fer a un intervalle de confiance d'environ 8,80 mg dans environ 70 % des globules rouges dans les unités incluses dans la prédiction.

8. Procédé selon l'une quelconque des revendications 1 à 7, dans lequel les données de l'étape a) sont obtenues par un dispositif d'enregistrement de données, tel que, par exemple, un lecteur de codes-barres.

9. Procédé selon l'une quelconque des revendications 1 à 8, comprenant en outre l'étape consistant à déterminer le fer total (mg) et/ou l'hémoglobine totale (g) dans ladite unité.

10. Procédé selon l'une quelconque des revendications 1 à 9, dans lequel ledit procédé est automatisé, tel que, par exemple, mis en œuvre par un robot.

11. Appareil de traitement de données comprenant un moyen pour mettre en œuvre le procédé des revendication 1 à 10.

12. Programme informatique comprenant des instructions qui, lorsque le programme est exécuté par un ordinateur, amènent l'ordinateur à mettre en œuvre le procédé de l'une quelconque des revendications 1 à 10.

13. Support lisible par ordinateur sur lequel est stocké le programme informatique de la revendication 12.

Figure 1

A

B

Figure 2

A

Figure 2 (continued)

B)

C)

Figure 3

A)

B)

C)

Figure 3 (continued)

D)

MLR_all_features
(Adjusted R²: 0.914)

MLR_feature_reduction
(Adjusted R²: 0.916)

Figure 4

A)

Figure 4 (continued)

B)

C)

Figure 5

A)

B)

C)

Figure 5 (continued)

D)

Figure 6

Figure 7

A)

B)

Figure 8

A)

Figure 8 (continued)

B)

Figure 9

A

B

fragment_main

Welcome to

*Code Red*

DRK Baden-Württemberg-Hessen

START

*- from blood to code -*

←   fragment_doc_or_pat

Email address
xyz@abc.com
11/30

Password
•••••
5/30

LOG IN AS PATIENT

LOG IN AS PHYSICIAN

REGISTER

C

D

←   fragment_registration

## Registration

Type patient ID

Email address

Type age

Type height (cm)

Type weight (cm)

Select ethnicity

Select diagnosis

Password

Confirm password

CONFIRM AND SEND

←   fragment_doc

Select patient ID

ADD HEMOGLOBIN AND IRON INPUT

ADD FERRITIN CONCENTRATION

ADD LIVER IRON CONCENTRATION

MONITOR IRON LEVELS

DISPLAY BLOOD GROUP

DISPLAY IRREGULAR ANTIBODIES

ADD IRON LOSS

Figure 9 (continued)

E

F

G

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 107356533 A
- CN 107367484 A

### Non-patent literature cited in the description

- Red blood cell supernatant increases activation and agonist-induced reactivity of blood platelets. **CZU-BAK-PROWIZOR KAMILA et al.** THROMBOSIS RESEARCH. ELSEVIER, 24 October 2020, vol. 196, 543-549
- Comparison of efficacy of packed red blood cell transfusion based on its hemoglobin content versus the standard transfusion practice in thalassemia major patients (HEMOCON study). **RAJA ARSHAD et al.** TRANSFUSION AND APHERESIS SCIENCE. ELSEVIER SCIENCE, 27 January 2020, vol. 59
- *Global Forum for Blood Safety: Patient Blood Management*, 2011, https://www.who.int/bloodsafety/events/gtbs_01_pbm/en
- **MÜLLER,M.M.** ; **GEISEN,C.** ; **ZACHAROWSKI,K.** ; **TONN,T.** ; **SEIFRIED,E.** Transfusion of Packed Red Cells. *Dtsch Arztebl Int*, 2015, vol. 112, 507-518
- **CHRISTOU,G. et al.** Optimal transfusion practices after allogeneic hematopoietic cell transplantation: a systematic scoping review of evidence from randomized controlled trials. *Transfusion*, 2016, vol. 56, 2607-2614
- **SHANDER,A.** ; **CAPPELLINI,M.D.** ; **GOODNOUGH,L.T.** Iron overload and toxicity: the hidden risk of multiple blood transfusions. *Vox Sang.*, 2009, vol. 97, 185-197
- **NEUKIRCHEN,J. et al.** Improved survival in MDS patients receiving iron chelation therapy - a matched pair analysis of 188 patients from the Dusseldorf MDS registry. *Leuk. Res.*, 2012, vol. 36, 1067-1070
- **REMACHA,A. et al.** Guidelines on haemovigilance of post-transfusional iron overload. *Blood Transfus.*, 2013, vol. 11, 128-139
- **HAGHPANAH,S.** ; **ZAREI,T.** ; **ZAHEDI,Z.** ; **KARIMI,M.** Compliance and satisfaction with deferasirox (Exjade(R)) compared with deferoxamine in patients with transfusion-dependent beta-thalassemia. *Hematology*, 2014, vol. 19, 187-191
- **ZURLO,M.G. et al.** Survival and causes of death in thalassaemia major. *Lancet*, 1989, vol. 2, 27-30
- **AGNIHOTRI,N.** ; **PAL,L.** ; **THAKUR,M.** ; **KUMAR,P.** The need to label red blood cell units with their haemoglobin content: a single centre study on haemoglobin variations due to donor-related factors. *Blood Transfus.*, 2014, vol. 12, 520-526
- Iron Overload and Chelation. **PORTER,J.** ; **VIPRAKASIT,V.** Guidelines for the Management of Transfusion Dependent Thalassaemia (TDT) [Internet]. Thalassaemia International Federation, 2014
- Neonatal and Pediatric Transfusion. **LAU,W.** Clinical Guide to Transfusion. Canadian Blood Services, 2017
- Principles of Red Blood Cell Transfusion. **NESS,P.M.** ; **KRUSKALL,M.S.** Hematology: Basic Principles and Practice. 2005
- **ALI,A.** ; **AUVINEN,M.K.** ; **RAUTONEN,J.** The aging population poses a global challenge for blood services. *Transfusion*, 2010, vol. 50, 584-588
- **GREINACHER,A.** ; **FENDRICH,K.** ; **HOFFMANN,W.** Demographic Changes: The Impact for Safe Blood Supply. *Transfus. Med. Hemother.*, 2010, vol. 37, 141-148
- **ARSLAN,O.** Hemosoft: a new software for blood bank and apheresis management. *Transfus. Apher. Sci.*, 2004, vol. 30, 193-196
- **ATILLA,E.** ; **TOPRAK,S.K.** ; **CIVRIZ,B.S.** ; **TOPCUOGLU,P.** ; **ARSLAN,O.** A Randomized Comparison of Hemoglobin Content-Based Versus Standard (Unit-Based) Red Blood Cell Transfusion Policy. *Turk. J. Haematol.*, 2017, vol. 34, 244-249
- **ESTEVA,A. et al.** Dermatologist-level classification of skin cancer with deep neural networks. *Nature*, 2017, vol. 542, 115-118
- **ANDERSON,J.P. et al.** Reverse Engineering and Evaluation of Prediction Models for Progression to Type 2 Diabetes: An Application of Machine Learning Using Electronic Health Records. *J. Diabetes Sci. Technol.*, 2015, vol. 10, 6-18
- **MCKINNEY,W.** Data Structures for Statistical Computing in Python. *Proceedings of the 9th Python in Science Conference. 9th Python in Science Conference. Proceedings of the 9th Python in Science Conference*, 2010, vol. 445, 51-56

- **HARRIS,C.R. et al.** Array programming with NumPy. *Nature*, 2020, vol. 585, 357-362
- **HUNTER,J.D.** Matplotlib: A 2D Graphics Environment. *Computing in Science & Engineering*, 2007, vol. 9, 90-95
- **WASKOM,W. et al.** *mwaskom/seaborn: v0.8.1*, September 2017, https://doi.org/10.5281/zenodo.883859. 2017
- **VIRTANEN,P. et al.** SciPy 1.0: fundamental algorithms for scientific computing in Python. *Nat. Methods*, 2020, vol. 17, 261-272
- TensorFlow: A system for large-scale machine learning. **ABADI,M. et al.** 12th USENIX Symposium on Operating Systems Design and Implementation (OSDI 16). USENIX Association, 2016, 265-283
- *scikeras 0.2.1*, https://pypi.org/project/scikeras/. 2020
- *Squarify*, https://pypi.org/project/squarify/. 2019
- **GANSNER,E.R. ; NORTH,S.C.** An open graph visualization system and its applications to software engineering. *Software Practice and Experience*, 2000, vol. 30, 1203-1233
- **VAN DER LAAN,M.J. ; POLLEY,E.C. ; HUBBARD,A.E.** Super learner. *Stat. Appl. Genet. Mol. Biol.*, 2007, vol. 6
- **POLLEY,E.C. ; VAN DER LAAN,M.J.** Super Learner In Prediction. Working Paper 266. *U.C. Berkeley Division of Biostatistics Working Paper Series*, 2010
- **FLENNERHAG,S.** *mlens Documentation; Release 0.1.6.*, https://mlens.readthedocs.io/ /downloads/en/0.1.x/pdf/. 2017
- **GUYON,I. ; ELISSEEFF,A.** An introduction to variable and feature selection. *Machine Learning*, 2003, vol. 3, 1157-1182
- **BREIMAN,L. ; FRIEDMAN,J. ; STONE,C.J. ; OLSHEN,R.A.** Classification and Regression Trees. Chapman and Hall/CRC, 1984
- **POTDAR,K. ; PARDAWALA,T.S. ; PAI,C.D.** A Comparative Study of Categorical Variable Encoding Techniques for Neural Network Classifiers. *International Journal of Computer Applications*, 2017, vol. 175, 7-9
- **FISCHLER,M.A. ; BOLLES,R.C.** Random sample consensus: a paradigm for model fitting with applications to image analysis and automated cartography. *Communications of the ACM*, 1981, vol. 24, 381-395
- **CORTES,C. ; VAPNIK,V.** Support-vector networks. *Machine Learning*, 1995, vol. 20, 273-297
- **BREIMAN,L.** Random Forests. *Machine Learning*, 2001, vol. 45, 5-32
- Discriminatory Analysis, Nonparametric Discrimination: Consistency Properties. **FIX,E. ; HODGES,J.L.** Technical Report. USAF School of Aviation Medicine, 1951, vol. 4
- LightGBM: a highly efficient gradient boosting decision tree. **KE,G. et al.** 31st International Conference on Neural Information Processing Systems (NIPS'17). Proceedings of the 31st International Conference on Neural Information Processing Systems (NIPS'17). Curran Associates Inc, 2017, 3149-3157
- **BRAUNITZER,G.** THE MOLECULAR WEIGHT OF HUMAN HAEMOGLOBIN. *Bibl. Haematol.*, 1964, vol. 18, 59-60
- **TRUDNOWSKI,R.J. ; RICO,R.C.** Specific gravity of blood and plasma at 4 and 37 degrees C. *Clin. Chem.*, 1974, vol. 20, 615-616
- **DAVENPORT,R.** Blood components should be labeled for content. *Transfusion*, 2005, vol. 45, 3-4
- **LO,B.D. et al.** Impact of body weight on hemoglobin increments in adult red blood cell transfusion. *Transfusion*, 2021
- **MUELLER,M.M. et al.** Patient Blood Management: Recommendations From the 2018 Frankfurt Consensus Conference. *JAMA*, 2019, vol. 321, 983-997
- **VOLKEN,T. et al.** Red blood cell use in Switzerland: trends and demographic challenges. *Blood Transfus.*, 2018, vol. 16, 73-82
- **BOLTON-MAGGS,P.H.B. ; WATT,A.** Transfusion errors - can they be eliminated?. *Br. J. Haematol.*, 2020, vol. 189, 9-20
- **DOAN,M. et al.** Objective assessment of stored blood quality by deep learning. *Proc. Natl. Acad. Sci. U. S. A*, 2020, vol. 117, 21381-21390
- **GEMAN,S. ; BIENENSTOCK,E. ; DOURSAT,R.** Neural Networks and the Bias/Variance Dilemma. *Neural Computation*, 1992, vol. 4, 1-58
- **ANSCOMBE,F.J.** Graphs in Statistical Analysis. *The American Statistician*, 1973, vol. 27, 17-21
- *DRK Hämotherapie*, 2019, https://www.drk-haemotherapie.de/ausgaben/ausgabe-32-2019
- Guidelines for the Management of Transfusion Dependent Thalassaemia (TDT). Thalassaemia International Federation, 2014
- **CAZZOLA,M. et al.** A moderate transfusion regimen may reduce iron loading in beta-thalassemia major without producing excessive expansion of erythropoiesis. *Transfusion*, 1997, vol. 37, 135-140
- **LIST,A.F. et al.** Deferasirox reduces serum ferritin and labile plasma iron in RBC transfusion-dependent patients with myelodysplastic syndrome. *J. Clin. Oncol.*, 2012, vol. 30, 2134-2139
- **ARSLAN,O. ; TOPRAK,S. ; ARAT,M. ; KAYALAK,Y.** Hb content-based transfusion policy successfully reduces the number of RBC units transfused. *Transfusion*, 2004, vol. 44, 485-488